# EUROPEAN PATENT SPECIFICATION

(11) **EP 2 671 079 B1**
(45) Date of publication and mention of the grant of the patent: **26.12.2018**
(21) Application number: 12705721.4
(22) Date of filing: 31.01.2012
(51) Int. Cl.: G01N 33/564, G01N 33/569, G01N 33/574

(54) **SNX9 AS A NOVEL BIOMARKER FOR CHRONIC INFLAMMATION AND ASSOCIATED IMMUNOSUPPRESSION AND A NEW REGULATOR OF T CELL RECEPTOR EXPRESSION AND FUNCTION**
SNX9 ALS NEUE BIOMARKER FÜR CHRONISCHE ENTZÜNDUNGEN UND ZUGEHÖRIGE IMMUNOSUPPRESSION SOWIE EIN NEUER REGULATOR DER T-ZELL-REZEPTOREXPRESSION UND FUNKTION
SNX9 EN TANT QUE NOUVEAU BIOMARQUEUR POUR L'INFLAMMATION CHRONIQUE ET L'IMMUNOSUPPRESSION ASSOCIÉE ET NOUVEAU RÉGULATEUR DE L'EXPRESSION DES RÉCEPTEURS DE CELLULES T ET FONCTION

(30) Priority: 31.01.2011 US 201161437952 P
(43) Date of publication of application: 11.12.2013
(73) Proprietor: Yissum Research Development Company of the Hebrew University of Jerusalem Ltd., 91390 Jerusalem (IL)
(72) Inventor: BANIYASH, Michal, 9075804 Mevaseret Zion (IL); ISH SHALOM, Eliran, 9675520 Jerusalem (IL)
(74) Representative: Fleuchaus, Andrea
(86) International application number: PCT/IL2012/000053
(87) International publication number: WO 2012/104836

(56) References cited:
- EP-A1- 1 927 659
- WO-A2-2006/094673
- SHI L ET AL: "Proteomic investigation of the time course responses of RAW 264.7 macrophages to infection with Salmonella enterica", INFECTION AND IMMUNITY 2009 AMERICAN SOCIETY FOR MICROBIOLOGY USA LNKD- DOI:10.1128/IAI.00063-09, vol. 77, no. 8, August 2009 (2009-08), pages 3227-3233, XP002672783, ISSN: 0019-9567
- DATABASE Geneseq [Online] 28 December 2007 (2007-12-28), "Human class I MHC molecule binding polypeptide, SEQ ID 205.", XP002672784, retrieved from EBI accession no. GSP:ANN55464 Database accession no. ANN55464 & US 2007/248628 A1 (KELLER LORRAINE H [US] ET AL) 25 October 2007 (2007-10-25)
- J. PARK ET AL: "SNX18 shares a redundant role with SNX9 and modulates endocytic trafficking at the plasma membrane", JOURNAL OF CELL SCIENCE, vol. 123, no. 10, 15 May 2010 (2010-05-15) , pages 1742-1750, XP55023615, ISSN: 0021-9533, DOI: 10.1242/jcs.064170
- ROHAN D. TEASDALE ET AL: "Insights into the PX (phox-homology) domain and SNX (sorting nexin) protein families: structures, functions and roles in disease", BIOCHEMICAL JOURNAL, vol. 441, no. 1, 1 January 2012 (2012-01-01), pages 39-59, XP55023622, ISSN: 0264-6021, DOI: 10.1042/BJ20111226

## Description

### FIELD OF THE INVENTION

The present invention relates to diagnostic and prognostic methods and kits for assessing chronic inflammation and associated immune-suppression. More particularly, the invention relates to the use of SNX9 (sorting nexin 9 protein) as a biomarker for chronic inflammation and associated immune-suppression, specifically, in subjects suffering from chronic inflammatory conditions.

### BACKGROUND OF THE INVENTION

The inventors have previously shown that chronic inflammation leads to T and NK cell immuno-suppression associated with T cell antigen receptor (TCR) ζ chain (CD247) down-regulation, as observed under humans and mouse pathological conditions such as cancer, autoimmune and infectious diseases. T cells under such conditions exhibit normal surface expression levels of an atypical TCR devoid of ζ chain. These receptors are functionally impaired as attested by their inability to lead to *in vitro* and *in vivo* TCR-mediated T cell proliferation, failure to clear influenza viral infection or reject implanted tumor cells ¹⁻³. NK cells are also immuno-suppressed as indicated by their inability to clear influenza virus and to reject transplanted allogeneic cells ¹⁻³. The inventors had also shown that myeloid-derived suppressor cells (MDSCs) generated during the course of chronic inflammation are responsible for ζ chain down-regulation (most likely leading to its degradation in the lysosome) and for T and NK cell immunosuppression ^{2, 3}. However, the T cell mechanisms/molecules affected by the inflammatory environment that lead to ζ chain down-regulation, are yet unknown.

Under normal conditions the mechanisms controlling TCR surface expression and function are important for the regulation of immune responses and the prevention of T cell hyperactivation as well as autoimmunity ⁴⁻⁷. While TCRs in resting T cells are continuously internalized and recycled to the surface ⁸⁻¹⁰, following antigen recognition TCRs are polarized, their endocytosis is increased and recycling reduced, leading to surface TCR down-modulation and targeting to degradation, probably attenuating cell signaling ¹¹⁻¹⁵. The selective expression of only fully assembled TCRs at the cell surface is dictated by ζ; when ζ is missing there are almost no cell surface-expressed TCRs ^{16, 17}. In contrast, under chronic inflammation, although ζ is degraded, the remaining TCR subunits are expressed on the surface. However, their *in vitro* and *in vivo* function is impaired, resulting in immuno-suppression ¹⁻³.

The present invention aimed at identifying the molecular mechanism(s) responsible for ζ chain targeting to degradation under chronic inflammatory conditions. As firstly presented by the present invention, the inventors now discovered that the sorting nexin 9 protein (SNX9) regulates TCR expression pattern and function under normal conditions. Moreover, the inventors also identified SNX9 novel characteristics and expression pattern during chronic inflammatory conditions, suggesting its being a key player in the abnormal TCR features and T cell dysfunction observed during chronic inflammation. The inventors also identified that SNX9 is being negatively affected by the chronic inflammatory environment in B cells. Based on the current studies disclosed herein, SNX9 is a newly discovered TCR regulator, which is also a sensor for chronic inflammation and associated immuno-suppression in T and B cells.

SNX9 is considered as a ubiquitously expressed protein, and in the past few years it has been identified as an important protein participating in fundamental cellular activities. SNX9 binds strongly to dynamin and is partly responsible for the recruitment of this GTPase to sites of endocytosis ^{18, 19}. SNX9 also has a high capacity to modulate the membrane and therefore participates in key processes leading to endocytosis. It has also becomes clear that SNX9 has the ability to activate the actin regulator N-WASP in a membrane-dependent manner to coordinate actin polymerization with vesicle release ²⁰. Moreover, it was demonstrated that WASP inducibly associates with SNX9 after TCR/CD28 co-stimulation. SNX9 binds via its SH3 domain to WASP, via its PX domain to PI3K-generated PtdIns(3,4,5)P3 and to the p85 adaptor and interacts with WASP, p85, CD28, and clathrin in endocytic vesicles after T cell stimulation ²¹. It is important to note that SNX9 belongs to a family of sorting nexins and more specifically to a subgroup that includes the SNX18 and SNX33, which share common structural and functional characteristics ²²⁻²⁴.

In the course of the studies of the present invention, aiming at characterizing the molecular components involved in targeting ζ chain to degradation under chronic inflammatory conditions, the inventors hypothesized that SNX9 could be involved in this process due its characteristics as a molecule affecting receptor endocytosis, membrane trafficking, protein sorting and degradation. The new observations disclosed herein, allocate a role for SNX9 in TCR regulation under normal conditions, as well as in pathological conditions characterized by chronic inflammation.

The present inventors are the first to show the regulation of SNX9 in resting and activated T cells under normal conditions and pathologies characterized by chronic inflammatory environments. Under normal conditions the invention demonstrate: a) An association between the TCR and SNX9, b) That SNX9 expression is required for normal TCR expression, c) That the absence of SNX9 from T cells negatively affects TCR cap-formation during activation that could have consequences on immunological synapse (IS) formation and T cell activation, d) That activation of isolated T cells leads to increased SNX9 expression levels, and e) That in addition to SNX9 expression in T cells it is also highly expressed in B cells.

Under pathological conditions the inventors demonstrate: a) An *in vivo* regulation of SNX9 and SNX18 during chronic inflammation by using a mouse model system for chronic inflammation-induced immunosuppression; Under these conditions, SNX9 and SNX18 expression is depleted both in T and B cells while under an acute immune response SNX9 and SNX18 expression is not affected, b) That the massive SNX9 down modulation under chronic inflammation correlates with elevated levels of Gr1⁺Mac1⁺ MDSCs, which do not express SNX9, c) SNX9 down-regulation, which characterizes chronic inflammation, is a reversible phenomenon; when chronic inflammation/disease is recovered, SNX9 expression levels return to normal, d) SNX9 down-regulation is mediated via MDSCs, and e) SNX9 down-regulation is observed in the peripheral blood and spleen in a model systems developed by the present inventors for chronic inflammation. Moreover, the inventors also show SNX9 down-regulation in other mouse models for pathologies characterized by chronic inflammation such as: chronic infection (*Leishmania donovani*), cancer (melanoma and colorectal carcinoma) and autoimmune diseases (rheumatoid arthritis). Moreover, the inventors also show SNX9 down-regulation in blood samples isolated from melanoma patients, demonstrating thereby the feasibility of using the method of the invention as a reliable diagnostic and prognostic test. These results point at the possible use of SNX9 as a biomarker for chronic inflammation and associated immunosuppression. In addition, it could be used for monitoring efficacy of a given therapy leading, directly or indirectly, to the recovery from the chronic inflammatory environment as in cases of cancer, infections and autoimmune diseases. Moreover, these results identify SNX9 as an important protein for TCR/CD3 endocytosis and highlight a unique mechanism that controls surface TCR expression and T cell function under normal and chronic inflammatory conditions.

Thus, one object of the invention is to provide diagnostic and prognostic methods for detecting and monitoring chronic inflammation and associated immune-suppression in a mammalian subject, using SNX9 as a biomarker.

Another object of the invention is the provision of methods for evaluating the efficacy of a treatment with a therapeutic agent given to a subject suffering from a chronic-inflammatory condition and of methods for monitoring and assessing responsiveness of a subject suffering from a chronic-inflammatory condition to such treatment which will affect the inflammatory state, using SNX9 as a biomarker.

Another objet of the invention is to provide a prognostic tool for detecting responders vs. non-responders to a given immune-based therapy. Moreover, to provide a prognostic tool for the regression or recurrence of the diseases, which is the source of stimuli activating the chronic inflammatory response.

Another objet of the invention is to provide kits for detecting and monitoring chronic inflammation and associated immune-suppression in a mammalian subject.

These and other objects of the invention will become apparent as the description proceeds.

### SUMMARY OF THE INVENTION

According to a first aspect, the invention relates to a diagnostic and prognostic method for detecting and monitoring chronic inflammation and associated immune-suppression in a mammalian subject. In certain embodiments, the method of the invention comprises the step of determining the level of expression of SNX9 (sorting nexin 9 protein) in a biological sample of said subject to obtain an expression value. It should be noted that wherein a lower expression value as compared to a predetermined standard expression value or to an expression value of SNX9 in a control sample, is indicative of a chronic inflammation and associated immune-suppression in said subject.

A second aspect of the invention relates to a method for evaluating the efficacy of a treatment with a therapeutic agent given to a subject suffering from a chronic-inflammatory condition. The method comprises the step of: The first step (a), involves determining the level of expression of SNX9 in a biological sample of the subject, to obtain SNX9 expression value in the tested sample. It should be noted that the sample should be obtained prior to initiation of said treatment. The second step (b) determining the level of expression of SNX9 in at least one other biological sample of the subject, to obtain SNX9 expression value in said sample It should be noted that the at least one other sample is obtained after initiation of said treatment. In the third step (c), comparing SNX9 expression value in the biological sample obtained in step (a), with at least one SNX9 expression value obtained in step (b). In certain embodiments, a higher SNX9 expression value in a sample obtained after initiation of said treatment according to step (b) as compared to the SNX9 expression value in a sample obtained prior to initiation of said treatment according to step (a) is indicative of successful therapy.

A third aspect of the invention relates to a diagnostic and prognostic kit for detecting and monitoring chronic inflammation and associated immune-suppression in a mammalian subject. The kit of the invention comprises: (a) detecting molecules specific for determining the level of expression of SNX9 in a biological sample; (b) detecting molecules specific for determining the level of expression of at least one control reference protein in a biological sample, further comprising: detecting molecules specific for determining the level of expression of SNX18 in a biological sample; detecting molecules specific for determining the level of expression of TCR zeta chain in a biological sample and detecting molecules for determining MDSCs population in a biological sample; wherein said detecting molecule is at least one of an isolated antibody that specifically recognizes and binds SNX18, an isolated antibody that specifically recognizes and binds TCR zeta chain and an isolated antibody that specifically recognizes and binds Gr1.

These and other aspects of the invention will become apparent by the hand of the following figures.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE FIGURES

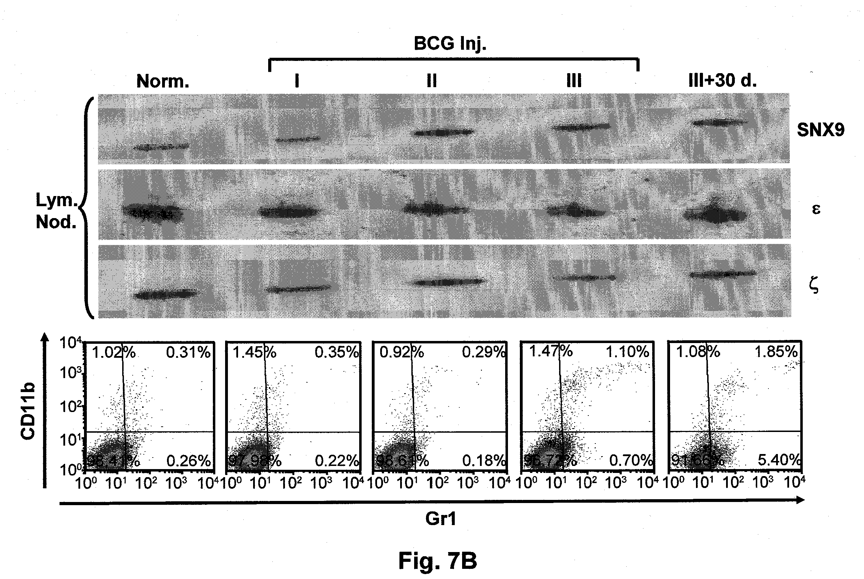

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.
**Fig 1A-1D****: SNX9 interacts with ζ chain**
   **Fig. 1A****.** Lysates prepared from EL4 T cell line were immuno-precipitated (IP) using the antibodies: anti-TCRβ, anti-CD3ε, anti-CD3δ and anti-ζ. Samples were subjected to immunoblotting with anti-SNX9 and anti-ζ antibodies.
   **Fig. 1B****.** Schematic representation of FL and truncated ζ-chain (proximal and distal). The extracellular (EC), transmembrane (TM), and cytoplasmic domains are depicted.
   **Fig. 1C****.** Extracts from COS-7 cells, which express endogenous SNX9, were transfected transiently with FL or truncated ζ-chain (proximal and distal). Cell lysates were immunoprecipitated with anti-SNX9 antibodies, and subjected to immunoblotting using antibodies against SNX9 or, ζ.
   **Fig. 1D****.** Extracts from COS-7 cells, which express endogenous SNX9, were transfected transiently with CD3ε. Cell lysates were immunoprecipitated with anti-SNX9 antibodies, and subjected to immunoblotting using antibodies against SNX9 or CD3ε.
      Abbreviations: Tot. (total), Lys. (lysate), FL (full length), Pro. (proximal), Dis. (distal), Transf. (transfection), WB (Western blot), TM (transmembrane), EC (extracellular), IP (immuno-precipitation), α (anti-).
**Fig 2A-2C****: SNX9 negatively regulates surface TCR expression and promotes TCR internalization after CD3 stimulation of EL4 cells**

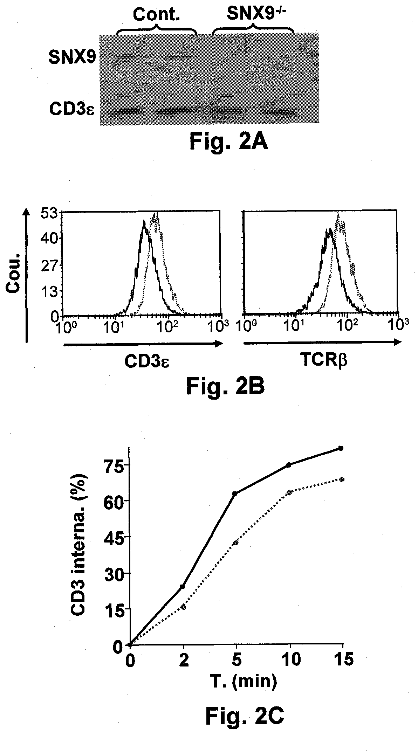

   **Fig.2A****.** Extracts from EL4 cells transduced with lentivectors encoding siRNA specific for SNX9 or an irrelevant target gene (Control) were resolved by SDS/PAGE and analyzed by immunoblotting with the indicated antibodies.
   **Fig. 2B****.** Control EL4 cells (black line) or SNX9 siRNA knockdown cells (dashed line) were stained for surface expression of CD3ε (left panel) and TCRβ (right panel) and analyzed by FACS.
   **Fig. 2C****.** Control EL4 cells (black line) or SNX9 siRNA knockdown cells (dashed line) were stimulated with PE conjugated anti-CD3ε antibodies at the indicated time points and analyzed for CD3 internalization using the ImageStream^{X} system.
      Abbreviations: Cont. (control), cou. (counts), interna (internalization), T (min) (time, minutes).

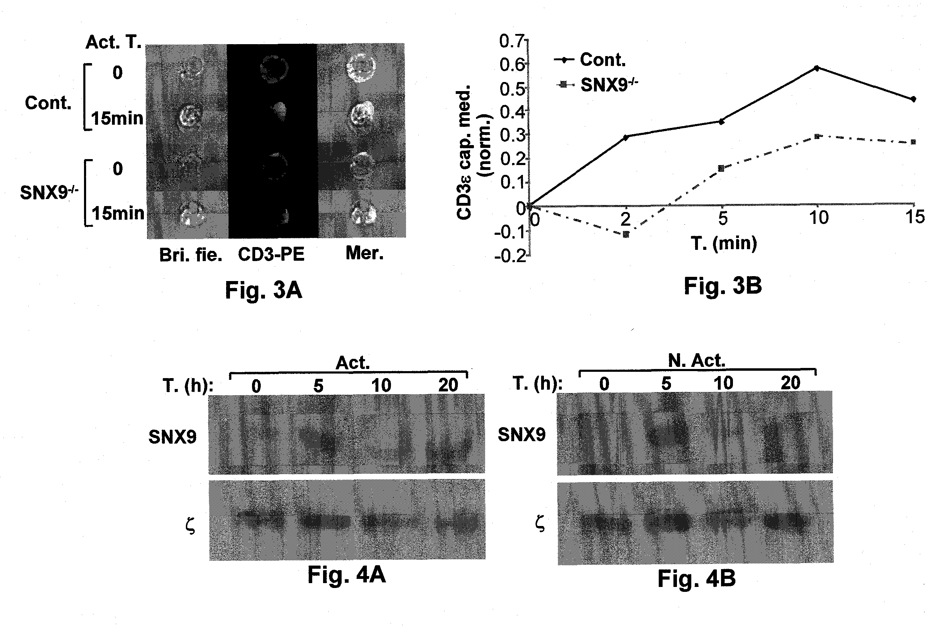

**Fig 3A-3B****. SNX9 negatively regulates TCR capping after CD3 stimulation of EL4 cells**
   **Fig. 3A****.** Control EL4 cells or SNX9 siRNA knockdown cells were stimulated with PE conjugated anti-CD3ε antibodies at the indicated time points and analyzed for CD3 cap formation using the ImageStream^{X} system. A typical representative image profile of CD3 cap formation after 15 min of stimulation vs. unstimulated cells is presented (the orange colored areas represent the volume of caped CD3 molecules).
   **Fig. 3B****.** A graph summarizes the average of 2000 cells at the indicated time points of CD3 capping of control EL4 cells (black line) or SNX9 siRNA knockdown cells (dashed line) (Lower panel).
      Abbreviations: cont. (control), Act. (activation), T (time), Bri. Fie. (bright field), Mer. (merge), Cap. Med. (capping median), norm. (normalized), min. (minutes).
**Fig 4A-4B****. SNX9 expression is increased in activated T cells and is down regulated in non-activated T cells incubated in tissue culture**
   **Fig. 4A****.** Splenic T cells were activated with CD3/CD28 antibodies at the indicated time points. Cell lysates were resolved by SDS/PAGE and subjected to immunoblotting using anti-SNX9 and anti-ζ antibodies.
   **Fig. 4B****.** Splenic T cells were incubated alone at the indicated time points. Cell lysates were resolved by SDS/PAGE and subjected to immunoblotting using anti-SNX9 and anti-ζ antibodies.
      Abbreviations: T (time), h (hour), Act. (activation), N. Act. (non-activation).

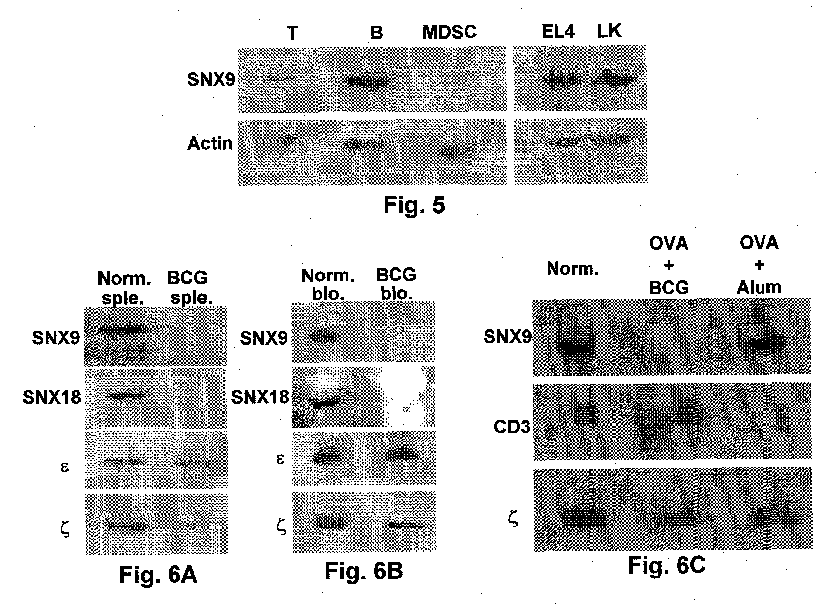

**Figure 5****. SNX9 is expressed in T and B cells but not in MDSC**
   SNX9 expression was tested in the following cell populations: freshly isolated T, B and MDSCs as well as in the cell lines EL4 (T cells) and LK (B cells). Cell were lysed, resolved on SDS/PAGE and subjected to immunoblotting using anti-SNX9 and anti-actin antibodies.
**Fig 6A-6E****. SNX9, SNX18 and ζ chain are down-regulated while SNX27 is stably expressed, upon BCG-induced chronic Th1 inflammatory response**
   **Fig. 6A****.** Splenic cells were isolated from BCG-treated and normal mice, lysed, resolved on SDS/PAGE and immuno-blotted with anti-SNX9, anti-SNX18, anti-ζ and anti-ε antibodies.
   **Fig. 6B****.** Blood cells were isolated from BCG-treated and normal mice, lysed, resolved on SDS/PAGE and immuno-blotted with anti-SNX9, anti-SNX18, anti-ζ and anti-ε antibodies.
   **Fig. 6C****.** Normal, OVA+BCG-treated (a Th1 response) or OVA+Alum treated (a Th2 response) mice were sacrificed and splenocytes from the three groups were analyzed by immunoblotting using anti-SNX9, anti-CD3ε and anti-ζ antibodies.

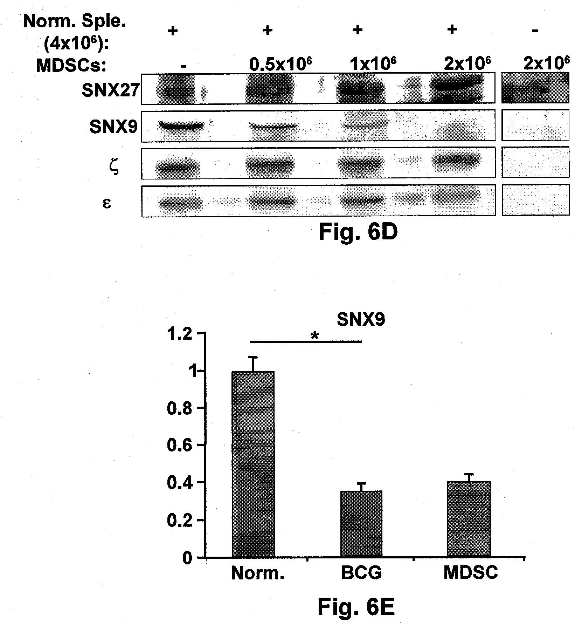

   **Fig. 6D****.** Splenic normal cells were co-incubated with rising amounts of splenic Gr1+Mac-1+ MDSCs (enriched 95% purity) isolated from mice treated with BCG for 18 hrs. Cells were then harvested, lysed, resolved on SDS/PAGE and analyzed by immunoblotting with anti-SNX27, anti-SNX9, anti-ζ and anti-CD3ε antibodies.
   **Fig. 6E****.** Graph presents levels of SNX9 mRNA from total splenic cells from normal mice, BCG treated mice and isolated MDSC's, as quantified by quantitative Real Time-PCR. Samples were normalized to the level of *Tubulin* mRNA. **P* < 0.05.
      Abbreviations: Norm. (normal), Sple. (spleen), Blo. (blood).

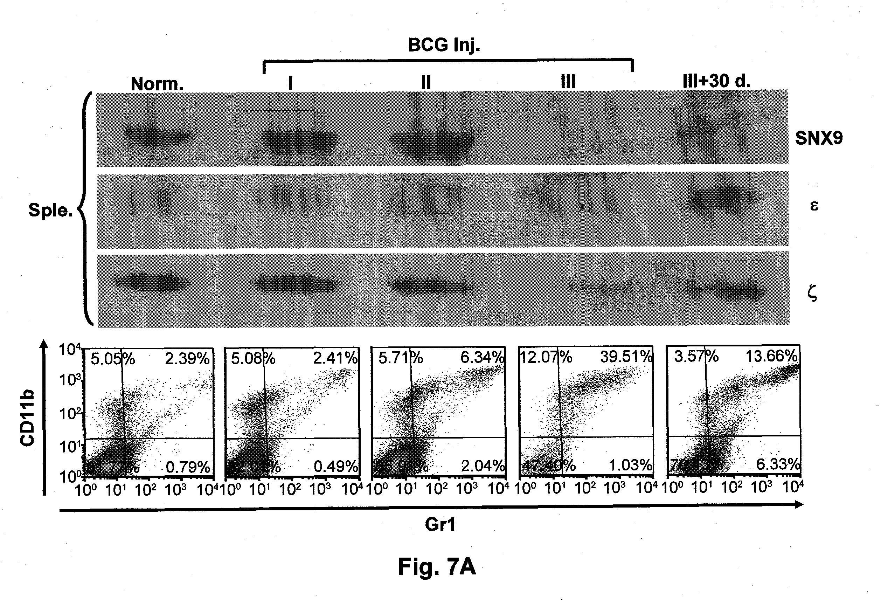

**Fig 7A-7B****. SNX9 down-regulation upon repeated BCG treatment correlates with elevated MDSCs levels-A reversible phenomenon**
   Spleens (**Fig. 7A**) and lymph nodes (**Fig. 7B**) were harvested from normal and treated mice, 3 days following the first (I), second (II) and third (III) BCG injections and after 30 days of recovery, following the third injection (III injection+30 days), as indicated. One part of the cells was subjected to lysis, resolved on SDS/PAGE and subjected to immunoblotting with anti-SNX9, anti-ζ chain and anti-CD3ε antibodies (upper panels) and the second part of the cells was double stained with specific antibodies for the detection of expanded Gr-1 and Mac-1 double-positive cells and subjected to FACS analysis (lower panels).
   Abbreviations: Sple. (spleen), Norm. (normal), Inj. (inject), d. (days), Lym. Nod. (lymph node).

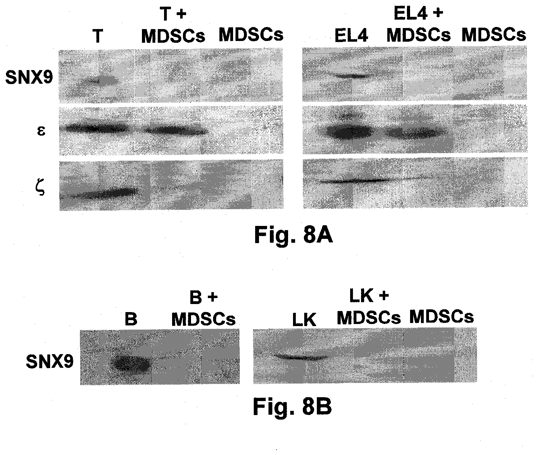

**Fig 8A-8B****. SNX9 down-regulation in T and B cells during chronic inflammation is MDSC-dependent**
   Isolated spleenic T and B cells from normal mice, EL4-T and LK-B cell lines were co-incubated with spleenic Gr1+Mac-1+ MDSCs (enriched 95% purity) isolated from BCG-treated mice in a ratio of 1:3 for 18 hrs. Cells were then harvested, lysed, resolved on SDS/PAGE and analyzed by immunoblotting with anti-SNX9, anti-ζ and anti-CD3ε antibodies.
   **Fig. 8A****.** Normal isolated T cells (left panel) and EL4 T cell line (right panel) were either co-incubated with cells from BCG-treated mice at ratio of or incubated alone.
   **Fig. 8B****.** Normal isolated B cells (left panel) and LK B cell line (right panel) were either co-incubated with cells from BCG-treated mice at ratio of or incubated alone.

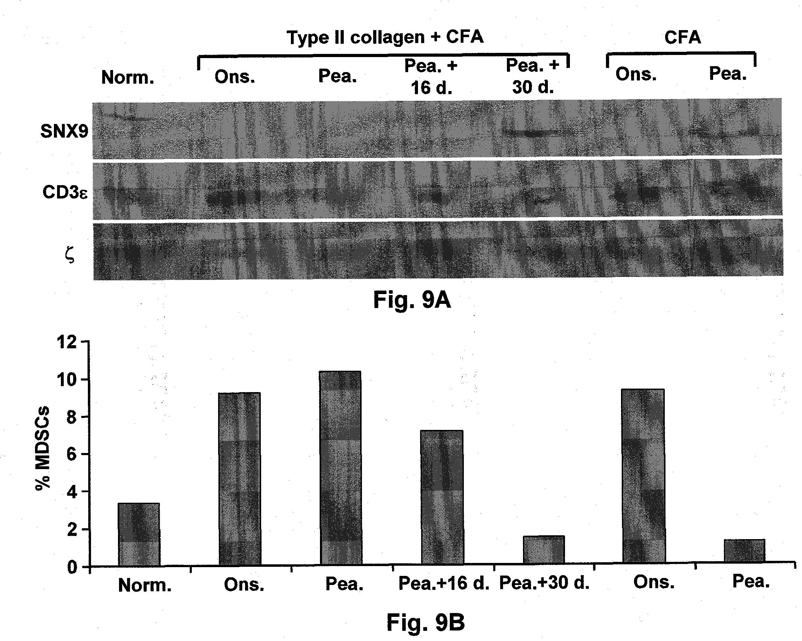

**Fig. 9A-9B****. SNX9 expression is down-regulated during experimental Collagen-Induced Arthritis (CIA)**
   **Fig. 9A****.** Splenic cells were harvested from normal mice and from mice at different stages of CIA developed upon injections with chicken type II collagen and CFA or from mice injected with CFA only, as indicated. The cell were lysed and resolved on SDS/PAGE and subjected to immunoblotting using anti-SNX9, anti-CD3ε and anti-ζ antibodies. Expression levels were compared with splenic cells isolated from CFA treated and normal mice.
   **Fig. 9B****.** Part of the cell suspension harvested from the different experimental groups in (A) was subjected to FACS analysis for Gr1⁺ Mac1⁺ double-positive cells.
      Abbreviations: Ons. (onset), pea. (peak), d. (days), Norm. (normal).

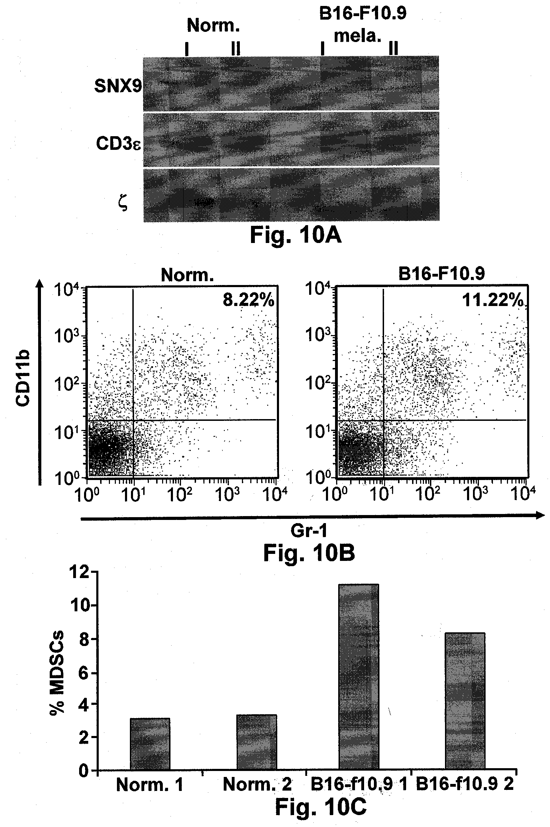

**Fig. 10A-10C****. B16-F10.9 melanoma induces SNX9 down-regulation**
   **Fig. 10A****.** Splenic cells were harvested from duplicate normal mice and from mice bearing subcutaneous B16-F10.9 melanoma, as indicated. The cells were lysed, resolved on SDS/PAGE and subjected to immunoblotting using anti-SNX9, anti-CD3ε and anti-ζ antibodies.
   **Fig. 10B****.** A representative FACS analysis of Gr1⁺Mac1⁺ double-positive cells of splenocytes isolated from normal mice or from subcutaneous B16-F10.9 melanoma-bearing mice.
   **Fig. 10C****.** Graphs summarizing FACS analysis profiles performed on duplicate mice in each experimental group for Gr1⁺Mac1⁺ double-positive cells from normal mice and mice bearing subcutaneous B16-F10.9 melanoma tumors.
      Abbreviations: Norm. (normal), mela. (melanoma), cont. (control).

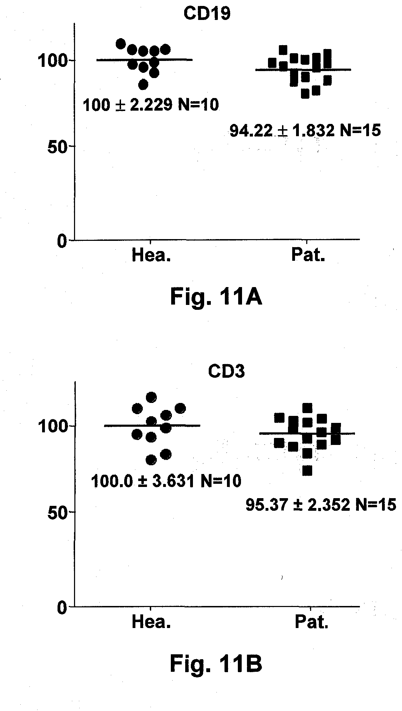

**Fig. 11A-11C****. SNX9 down-regulation in melanoma patients**
   **Figs 11A and 11B****.** Whole blood samples from healthy and melanoma donors were stained for total CD19 (B cells **Fig. 11A**) and CD3 (T cells, **Fig. 11B**) expression levels and measured by FACS. The results are presented as the mean fluorescent intensity (MFI). Each point in the graph represents the expression score of the indicated proteins. The score notes as values of each protein expression level normalized to its average expression in healthy donors. *P < 0.05

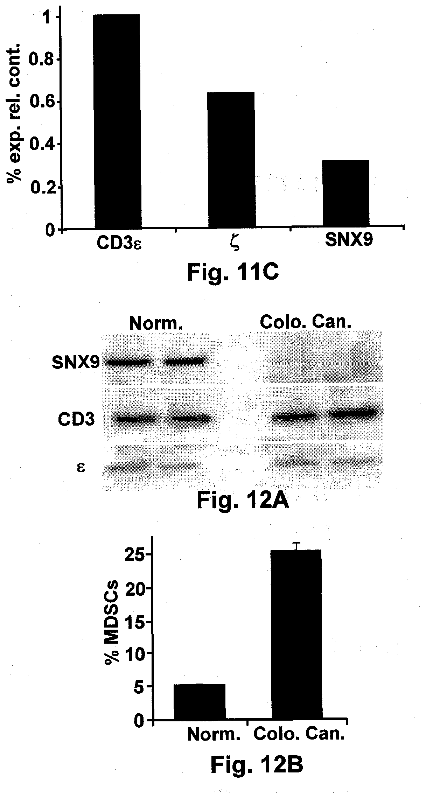

   **Fig. 11C****.** Graphs summarizing Western-blot analysis profiles performed on triplicate donors in each experimental group for the protein expression levels of CD3ε, ζ chain and SNX9 in the peripheral blood of the same healthy donors and melanoma patients used in Figs 11A-11B above. The results are presented as relative average values of each protein expression level normalized to its expression in healthy donors.
      Abbreviation: Hea. (healthy), Pat. (patients), exp. rel. cont. (percent expression relative to control).
**Fig. 12A-12B****. Colorectal cancer induces SNX9 down-regulation**
   **Fig. 12A****.** Splenic cells were harvested from normal and from tumor-bearing mice after AOM and DSS treatment, as indicated. The cells were lysed, resolved on SDS/PAGE and subjected to immunoblotting using anti-SNX9, anti-CD3ε and anti-ζ antibodies. A representative experiment is presented. While the ζ chain under these conditions is slightly down regulated if normalized to the CD3ε expression, the SNX9 expression levels are dramatically decreases.
   **Fig. 12B****.** Graphs summarizing FACS analysis profiles performed on triplicate mice in each experimental group for Gr1+Mac1+ double-positive MDSC cells from normal mice and from mice bearing colorectal tumor.
      Abbreviations: Norm. (normal), colo. can. (colorectal cancer).

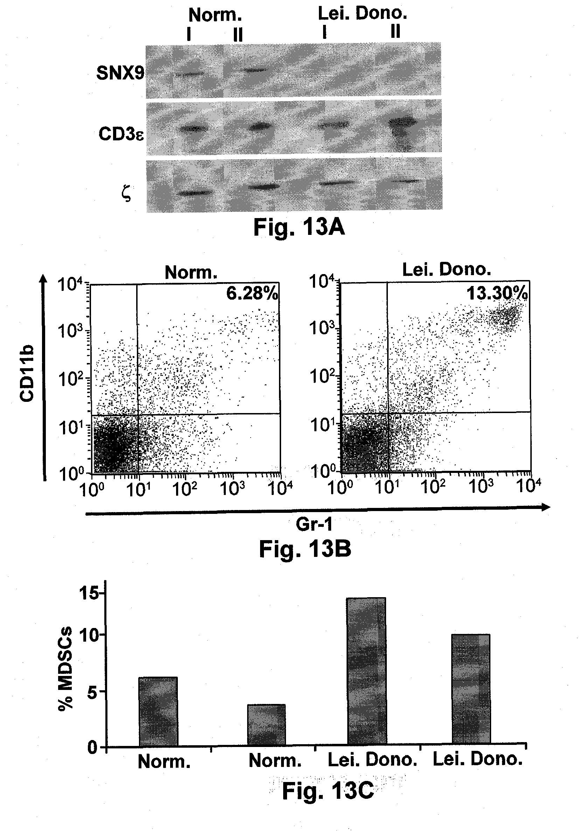

**Fig. 13A-13C****. Leishmania donovani infection induces SNX9 down-regulation**
   **13A.** splenic cells were harvested from duplicate normal and *L.donovani* infected mice, as indicated. The cell were lysed, resolved on SDS/PAGE and subjected to immunoblotting using anti-SNX9, anti-CD3ε and anti-ζ antibodies.
   **13B.** FACS analysis of Gr1⁺ Mac1⁺ double-positive cells from normal or L. donovani infected mice.
   **13C.** Graphs summarizing FACS analysis profiles of Gr1⁺ Mac1⁺ double-positive cells from duplicate normal and from *L. donovani* infected mice.
      Abbreviations: Norm. (normal), Lei. Don. (*L.donovani*).

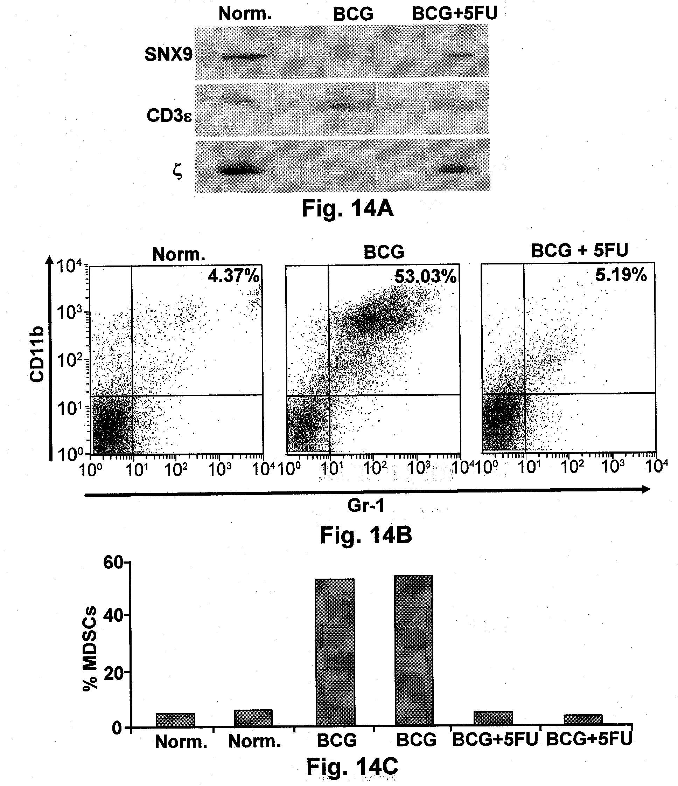

**Fig. 14A-14C****. SNX9 up-regulated following 5FU treatment**
   **Fig. 14A****.** Splenic cells were harvested from normal, BCG or BCG + 5FU treated mice, as indicated. The cell were lysed, resolved on SDS/PAGE and subjected to immunoblotting using anti-SNX9, anti-CD3ε and anti-ζ antibodies.
   **Fig. 14B****.** A representative FACS analysis of Gr1⁺ Mac1⁺ double-positive cells isolated from the three groups of mice.
   **Fig. 14C****.** Graphs summarizing FACS analysis profiles of Gr1⁺ Mac1⁺ double-positive cells from mouse duplicates of the three groups.
      Abbreviations: Norm. (normal).

### DETAILED DESCRIPTION OF THE INVENTION

The regulation of SNX9 in resting and activated T cells under normal conditions and in pathologies characterized by chronic inflammation is shown for the first time by the present invention.

The results presented herein identify SNX9 as an important protein for TCR/CD3 endocytosis and illustrate a unique mechanism for the control of surface TCR expression and T cell function under normal conditions. More importantly, the novel data of the invention point at the potential use of measuring SNX9 expression levels as a biomarker for determining the immune status in pathologies characterized by chronic inflammation, such as autoimmune diseases, proliferative disorders and during infections. Due to the reversible features of SNX9 down-regulation depending on the severity of the inflammatory environment, evaluation of SNX9 expression levels could serve as a biomarker for measuring the host's immune status as well as the efficacy of a given therapy, whether it directly or indirectly leads to the recovery of the disease and accordingly of the chronic inflammatory environment. Moreover, preliminary data presented by the present invention also show the similarity between SNX9 and SNX18 with regards to their sensitivity to the chronic inflammatory environment, indicating the potential use of SNX18, optionally, in combination with SNX9, as an additional biomarker for chronic inflammation.

Thus, according to a first aspect, the invention relates to a diagnostic and prognostic method for determining the immune status of a mammalian subject. More specifically, the invention provides diagnostic and prognostic method for detecting and monitoring chronic inflammation and associated immune-suppression in a mammalian subject. In certain embodiments, the method of the invention comprises the step of determining the level of expression of SNX9 (sorting nexin 9 protein) in a biological sample of a subject to obtain an expression value. It should be noted that wherein a lower expression value as compared to a predetermined standard expression value or to an expression value of SNX9 in a control sample, is indicative of a chronic inflammation and associated immune-suppression in the diagnosed subject.

The method of the invention provides the use of SNX9 as a biomarker for chronic inflammation. It should be appreciated that in certain embodiments, as used herein in the specification and in the claim section below, SNX9 protein refers to the human SNX9 (sorting nexin 9 protein). More specifically, the human SNX9 protein comprises an amino acid sequence of 595 amino acid residues as denoted by GenBank Accession No. NP_057308.1 GI: 7706706, encoded by a nucleic acid sequence of 4,200 bp linear mRNA, as denoted by Accession: NM_016224.3 GI: 23111056. It should be further appreciated that the Homo sapiens SNX9, Gene ID as indicated by the NCBI Gene database, is 51429.

Still further, the invention further refers (specifically in the Examples section) in some particular embodiments to sorting nexin-9 [Mus musculus] that is a 595 amino acids long protein having the Accession: NP_079940.2 GI: 29568084. Mus musculus SNX9 mRNA comprises 2,078 bp linear mRNA, and is denoted by Accession: NM025664.5 GI: 153791678. The mouse SNX9 Gene ID as indicated by the NCBI Gene database is 66616.

As indicated above, the method of the invention is intended for determining the immune state of a mammalian subject, specifically, detecting chronic inflammation and associated immune-suppression in the subject. Chronic inflammatory state, as used herein is reflected by an inflammatory response. As used herein the phrase "inflammatory response" refers to an immune response which results in either chronic or acute inflammation, typically occurring as a result of injurious stimuli including infection, burns, trauma, neoplasia, autoimmune signals and exposure to chemicals, heat or cold or any other harmful stimulus. An inflammatory response according to the present invention refers to a chronic inflammation.

As indicated above, the diagnostic and prognostic method of the invention is based on determining the expression level of a specific biomarker, SNX9, in a sample. The terms "level of expression" or "expression level" are used interchangeably and generally refer to the amount of a polynucleotide or an amino acid product or protein in a biological sample. "Expression" generally refers to the process by which gene-encoded information is converted into the structures present and operating in the cell. Therefore, according to the invention "expression" of a gene, specifically, a gene encoding SNX9 may refer to transcription into a polynucleotide, translation into a protein, or even posttranslational modification of the protein. Fragments of the transcribed polynucleotide, the translated protein, or the post-translationally modified protein shall also be regarded as expressed whether they originate from a transcript generated by alternative splicing or a degraded transcript, or from a post-translational processing of the protein, e.g., by proteolysis. It should be noted that the expression level is reflected by measurement and determination of an expression value. As used herein, the term "expression value", "level of expression" or "expression level" refers to numerical representation of a quantity of a gene product, which herein is a protein, but may also be an mRNA.

As shown by the present invention as disclosed in the following Examples, reduced, or lower expression of SNX9 is correlated with a chronic inflammation status. "Low level of expression", as used herein for SNX9, denotes a level significantly (e.g. as determined by statistical determination) lower than a standard. "Standard" or a "predetermined standard" as used herein, denotes either a single standard value or a plurality of standards with which the level of SNX9 expression from the tested sample is compared. The standards may be provided, for example, in the form of discrete numeric values or is calorimetric in the form of a chart with different colors or shadings for different levels of expression; or they may be provided in the form of a comparative curve prepared on the basis of such standards. The standards may be prepared by determining the level of expression of SNX9 present in a sample obtained from a plurality of patients positively diagnosed (by other means, for example by a physician, by histological techniques etc.) as having a chronic inflammatory condition at varying levels of severity (being correlated with level of expression of SNX9). The level of expression for the preparation of the standards may also be determined by various conventional methods known in the art. The methods of the invention may be carried out in parallel to a number of standards of healthy subjects and subjects of different chronic inflammatory condition states and the level determined in the assayed sample is then compared to such standards. After such standards are prepared, it is possible to compare the level of SNX9 expression obtained from a specific tested subject to the corresponding value of the standards, and thus obtain an assaying tool.

More specifically, in certain embodiments, wherein indicated "lower", "reduced" or "decreased" expression levels of SNX9, it is meant that such decrease or reduction may be a decrease or reduction of between about 10% to 100% of the expression of such biomarker. The terms "decrease", "reduction" and "elimination" as used herein relate to the act of becoming progressively smaller in size, amount, number, or intensity. Particularly, a reduction of 10%, 15%, 20%, 25%, 30%, 35%, 40%, 45%, 50%, 55%, 60%, 65%, 70%, 75%, 80%, 85%, 90%, 95% or 100% of the expression as compared to a suitable control. It should be further noted that decrease or reduction may be also a decrease of about 2 to 100 folds. Still further, it should be appreciated that the decrease of the levels or expression of said SNX9 may be either in the transcription, translation or the stability of said biomarker. With regards to the above, it is to be understood that, where provided, percentage values such as, for example, 10%, 50%, 120%, 500%, etc., are interchangeable with "fold change" values, i.e., 0.1, 0.5, 1.2, 5, etc., respectively.

According to one specific embodiment, determining the level of expression of SNX9 in a biological sample of a subject according to the diagnostic method of the invention may be performed by the following steps:
The first step (a) involves contacting detecting molecules specific for SNX9 with a biological sample (or with an aliquot thereof) of said subject, or alternatively, with any protein or nucleic acid product obtained from the sample. It should be noted that optionally, the detecting molecules may be contacted with a control sample or with any protein or nucleic acid product obtained therefrom.

In a second step (b), contacting detecting molecules specific for at least one reference control, with a biological sample of said subject (or with an aliquot thereof), or with any protein or nucleic acid product obtained therefrom. Optionally, the control reference detecting molecules are contacted with a control sample or with any protein or nucleic acid product obtained therefrom.

A third step of the method (c) requires determining the expression value of SNX9 in the biological sample according to step (a), and optionally, in a suitable control sample.

The forth step (d), requires determining the expression value of said at least one reference control in the biological sample according to step (b), and optionally, in a suitable control sample.

The next step (e) involves comparing the SNX9 expression value in the test biological sample obtained in step (c), with a predetermined standard expression value, or a cutoff value, or with an expression value of SNX9 in a control sample optionally obtained in step (c). It should be noted that in certain embodiments, a lower expression value of SNX9 in the tested sample, as compared to a predetermined standard expression value or to the expression value of SNX9 in a control sample, is indicative of a chronic inflammation and associated immune-suppression in the tested subject.

According to certain and specific embodiments, the method of the invention further comprises an additional and optional step of normalization. According to this embodiment, after determination of the expression levels of SNX9 and the reference control in steps (c) and (d), respectivelly, the expression value of the biomarker SNX9 obtained in step (c) is normalized according to the expression value of said at least one reference control obtained in step (d), in the test sample. Optionally, similar normalization is performed also relative to a control sample or a representing standard when applicable. According to this particular embodiment, a normalized expression value of SNX9 in the test sample, and optionally relative to a control sample is obtained. The next step in this embodiment, involves comparing the normalized SNX9 expression value in the test biological sample obtained in this additional step, with a predetermined standard expression value, or a cutoff value, or with a normalized expression value of SNX9 in a control sample optionally obtained in this additional step. It should be noted that in certain embodiments, a lower expression value of SNX9 in the tested sample, as compared to a predetermined standard expression value or to the expression value of SNX9 in a control sample, is indicative of a chronic inflammation and associated immune-suppression in the tested subject.

As indicated above, the first step of the diagnostic method of the invention involves contacting the sample or any aliquot thereof with detecting molecules specific for SNX9. The term "contacting" means to bring, put, incubate or mix together. As such, a first item, that is the sample or any nucleic acids or amino acids obtained therefrom, is contacted with a second item, that is the detecting molecule, when the two items are brought or put together, e.g., by touching them to each other or combining them. In the context of the present invention, the term "contacting" includes all measures or steps which allow interaction between the detection molecules for SNX9, or for at least one suitable control reference protein (for example, CD3ε for T cells, CD19 for B cells or SNX27 for both) and the tested sample or any nucleic acids or proteins obtained therefrom. The contacting is performed in a manner so that the at least one of detecting molecule of SNX9 and at least one suitable control reference protein or nucleic acid molecule can interact with or bind to the protein or nucleic acid molecules in the tested sample. The binding will preferably be non-covalent, reversible binding, e.g., binding via salt bridges, hydrogen bonds, hydrophobic interactions or a combination thereof.

After contacting the sample with the specific detecting molecules, the expression values of SNX9 and of the reference control are determined in steps (c) and (d), by a suitable means, as will be explained herein after.

As mentioned above, the optional normalization step of the method of the invention involves normalization of the measured expression values of SNX9, to obtain normalized expression value. As indicated herein, the measured levels of expression of SNX9 are routinely normalized using data of expression levels of the control reference proteins. In general scientific context, normalization is a process by which a measurement raw data is converted into data that may be directly compared with other so normalized data. In the context of the present invention, measurements of marker genes or proteins, expression levels are prone to errors caused by, for example, unequal degradation of measured samples, different loaded quantities per assay, and other various errors. More specifically, any assayed sample may contain more or less biological material than is intended, due to human error and equipment failures. Thus, the same error or deviation applies to both the biomarker of the invention and to the control reference, whose expression is essentially constant. Thus, division of the SNX9 raw expression value by the control reference raw expression value yields a quotient which is essentially free from any technical failures or inaccuracies (except for major errors which destroy the sample for testing purposes) and constitutes a normalized expression value of said biomarker. This normalized expression value may then be compared with normalized cutoff values, i.e., cutoff values calculated from normalized expression values. Since control reference expression values are equal in different samples, they constitute a common reference point that is valid for such normalization. More specifically, in the present case, the expression of control references used by the invention, i.e., CD3ε for T cells, CD19 for B cells or SNX27 for both, is equal and stable in samples displaying chronic inflammation and in control samples of healthy donors that do not display chronic inflammation. Therefore, reduction in the expression of SNX9 in a test sample, vs. stable expression of the control reference in the same sample or vs. it expression in healthy donors samples, indicates that the sample is of a subjects having chronic inflammation.

As described hereinabove, step (e) of the method for the detection and monitoring of chronic inflammation provided by the invention, refers to a predetermined cutoff value or predetermined standard expression value. It should be noted that a "cutoff value", sometimes referred to simply as "cutoff' herein, is a value that meets the requirements for both high diagnostic sensitivity (true positive rate) and high diagnostic specificity (true negative rate). SNX9 expression level values that are higher or lower in comparison with said SNX9 expression corresponding cutoff value or a predetermined standard value indicate that the examined sample belongs to a pre-established population associated with a specific chronic inflammation rate (low or high, respectively) and limited to the said sensitivity and specificity.

It should be noted that the terms "sensitivity" and "specificity" are used herein with respect to the ability of the biomarker of the invention, SNX9, to correctly classify a sample as belonging to a pre-established population associated with a specific chronic inflammation states.

"Sensitivity" indicates the performance of the biomarker of the invention, SNX9, with respect to correctly classifying samples as belonging to pre-established populations that are likely to display a chronic inflammation status. In case where said SNX9 expression values are lower than the cutoff, that is, positive values indicating higher chronic inflammation states, more specifically, indicating that the diseased subject is more likely to display a chronic inflammation than corresponding pre-established populations wherein said corresponding SNX9 expression values are higher than the cutoff, that is, negative values indicating lower chronic inflammation rates.

"Specificity" indicates the performance of the biomarker, SNX9, with respect to correctly classifying samples as belonging to pre-established populations that are unlikely to display a chronic inflammation and associated immuno-suppression state, wherein said SNX9 expression values are higher than the cutoff, that is, negative values indicating lower chronic inflammation rates less likely to display inflammation than corresponding pre-established populations wherein said corresponding SNX9 expression values are lower than the cutoff, that is, positive values indicating higher inflammation rates.

Simply put, "sensitivity" relates to the rate of correct identification of high-chronic inflammation rate samples as such out of a group of samples, whereas "specificity" relates to the rate of correct identification of low-inflammation rate samples as such out of a group of samples, in a reproducible manner.

Cutoff values may be used as a control sample, said cutoff values being the result of a statistical analysis of SNX9 expression value differences in pre-established populations with either a chronic inflammation state or "healthy" subjects with no inflammation. Specifically, it is understood that SNX9 expression values lower than the cutoff value found by the inventors (i.e., positive expression value), indicate a higher tendency for chronic inflammation in a patient than a patient where the corresponding SNX9 expression values are higher than the cutoff value (i.e., negative results). Thus, a given population having specific clinical parameters will have a defined likelihood to have chronic inflammation based on the expression values of SNX9 above or below said cutoff values. It should be emphasized that the nature of the invention is such that the accumulation of further patient data may improve the accuracy of any obtained cutoff values, which are usually based on an ROC (Receiver Operating Characteristic) curve generated according to accumulated patient data using, for example, a commercially available analytical software program. The SNX9 expression values are selected along the ROC curve for optimal combination of diagnostic sensitivity and specificity, which are as close to 100% as possible, and the resulting values are used as the cutoff values that distinguish between patients who will display chronic inflammation at a certain rate, and those who will not (with said given sensitivity and specificity). The ROC curve may evolve as more and more patient-chronic inflammation data and related SNX9 expression values are recorded and taken into consideration, modifying the optimal cutoff values and improving sensitivity and specificity. Thus, any cutoff values should be viewed as a starting point that may shift as more patient-chronic inflammation data allows more accurate cutoff value calculation.

More specifically, and as explained earlier, the inventors have analyzed the expression values of SNX9 further, in order to discover specific cutoff values, a deviation from which is indicative of an increased likelihood for chronic inflammation and immuno-suppression state in a tested subject that suffers from a chronic inflammatory condition. It should be appreciated that an important step in the diagnostic and prognostic method of the invention is determining whether the normalized expression value of SNX9 is positive and thereby belongs to a pre-established population with an associated specific chronic inflammation state, or is negative and thereby belongs to a pre-established population with a different specific chronic inflammation state. The presence of at SNX9 with a positive normalized expression value indicates that the subject belongs to a pre-established population with an associated chronic inflammation state which is higher than the chronic inflammation state associated with, *ceteris paribus,* subjects where SNX9 have negative normalized expression values, "positive" and "negative" referring to the relation of said expression values to said cutoff value. According to certain embodiments a "positive result" may be determined where a normalized value of SNX9 is lower than the cutoff value and therefore predicts chronic inflammation and associated immuno-suppression.

More specifically, as used herein the phrase "a decrease below a predetermined cutoff or threshold" refers to a decrease in the ratio determined in the sample of the chronic inflammatory condition relative to the reference ratio which is lower than a predetermined cutoff or threshold such as about 10 %, e.g., lower than about 20 %, e.g., lower than about 30 %, e.g., lower than about 40 %, e.g., lower than about 50 %, e.g., lower than about 60 %, lower than about 70 %, lower than about 80 %, lower than about 90 %, lower than about 2 times, lower than about three times, lower than about four time, lower than about five times, lower than about six times, lower than about seven times, lower than about eight times, lower than about nine times, lower than about 20 times, lower than about 50 times, lower than about 100 times, lower than about 200 times, lower than about 350, lower than about 500 times, lower than about 1000 times, or more relative to the reference ratio. According to some embodiments of the invention, an identical ratio or a change above a predetermined cutoff or threshold in the ratio determined in the sample of the chronic inflammatory condition as compared to the reference ratio indicates the absence of the chronic inflammation.

As shown by Example 2, similarly to SNX9, the expression of SNX18 is also correlated with chronic inflammation. Moreover, Example 3 discloses a correlation between increased population of myeloid derived suppressor cells (MDSCs), reduced expression of SNX9, and chronic inflammation state. Still further, using a melanoma model, Example 6 shows that TCRζ chain expression is sensitive to recovery of inflammatory environment, wherein the expression levels of SNX9 are correlated with changes in the inflammatory environment. Taken together, these data form a basis for the combined use of SNX9 with other biomarkers such as SNX18, TCRζ chain and MDSCs population.

Thus, in certain specific embodiments, the method of the invention may combine other biomarkers, and therefore may further comprise at least one of the following steps:
(a) determining the level of expression of SNX18 (sorting nexin 18 protein) in a biological sample of said subject, to obtain an expression value; (b) determining the level of expression of T cell antigen receptor (TCR) ζ chain in a biological sample of said subject, to obtain an expression value; and (c) determining myeloid-derived suppressor cells (MDSCs) population in a biological sample of said subject.

It should be noted that a lower expression value of SNX9 and of at least one of SNX18 and TCR ζ chain as compared to a predetermined standard expression values or to the expression values of SNX9 and optionally, of at least one of SNX18 and TCR ζ chain in a control sample, indicate a chronic inflammation and associated immune-suppression in said subject. In yet another embodiment, the method of the invention may further comprise, in addition to determination of SNX9 expression levels, the determination of MDSCs population in the sample. In such case, reduced expression of SNX9 with a detected increase in the population of MDSCs indicates that the tested subject is in chronic inflammatory state.

It should be appreciated that determination of the level of SNX9 expression in the biological sample can be effected at the transcriptional level (i.e., mRNA) using detecting molecules that are based on nucleic acids (an oligonucleotide probe or primer), or alternatively, at the translational level (i.e. protein) using amino acid based detecting molecules, as also demonstrated by the present invention. Thus, according to one specific embodiment, the detecting molecules used by the diagnostic method of the invention may be isolated detecting amino acid molecules or isolated detecting nucleic acid molecules, or any combinations thereof.

According to one specific embodiment, the detection of SNX9 expression can be effected at the protein level. Therefore, the detecting molecules used by the method of the invention may be amino acid molecules, specifically, an isolated antibodies that specifically recognize and binds SNX9.

As indicated above, the detecting molecules of the invention may be amino acid based molecules that may be referred to as protein/s or polypeptide/s. As used herein, the terms "protein" and "polypeptide" are used interchangeably to refer to a chain of amino acids linked together by peptide bonds. In a specific embodiment, a protein is composed of less than 200, less than 175, less than 150, less than 125, less than 100, less than 50, less than 45, less than 40, less than 35, less than 30, less than 25, less than 20, less than 15, less than 10, or less than 5 amino acids linked together by peptide bonds. In another embodiment, a protein is composed of at least 200, at least 250, at least 300, at least 350, at least 400, at least 450, at least 500 or more amino acids linked together by peptide bonds. It should be noted that peptide bond as described herein is a covalent amid bond formed between two amino acid residues.

The invention further contemplates the use of amino acid based molecules such as proteins or polypeptides as detecting molecules disclosed herein and would be known by a person skilled in the art to measure the protein products of the marker genes of the invention. Techniques known to persons skilled in the art (for example, techniques such as Western Blotting, Immunoprecipitation, ELISAs, protein microarray analysis, Flow cytometry and the like) can then be used to measure the level of protein products corresponding to the biomarker of the invention. As would be understood to a person skilled in the art, the measure of the level of expression of the protein products of the biomarker of the invention, specifically, SNX9 requires a protein, which specifically and/or selectively binds to the biomarker genes of the invention.

In specific embodiments, the detecting amino acid molecules are isolated antibodies, with specific binding selectively to SNX9. Using these antibodies, the level of expression of SNX9 may be determined using an immunoassay which is selected from the group consisting of FACS, a Western blot, an ELISA, a RIA, a slot blot, a dot blot, immunohistochemical assay and a radio-imaging assay.

The term "antibody" as used in this invention includes whole antibody molecules as well as functional fragments thereof, such as Fab, F(ab')2, and Fv that are capable of binding with antigenic portions of the target polypeptide, i.e. SNX9. The antibody is preferably monospecific, e.g., a monoclonal antibody, or antigen-binding fragment thereof. The term "monospecific antibody" refers to an antibody that displays a single binding specificity and affinity for a particular target, e.g., epitope. This term includes a "monoclonal antibody" or "monoclonal antibody composition", which as used herein refer to a preparation of antibodies or fragments thereof of single molecular composition.

It should be recognized that the antibody can be a human antibody, a chimeric antibody, a recombinant antibody, a humanized antibody, a monoclonal antibody, or a polyclonal antibody. The antibody can be an intact immuno globulin, e.g., an IgA, IgG, IgE, IgD, 1gM or subtypes thereof. The antibody can be conjugated to a functional moiety (e.g., a compound which has a biological or chemical function. The antibody used by the invention interacts with a polypeptide that is SNX9, with high affinity and specificity.

As noted above, the term "antibody" also encompasses antigen-binding fragments of an antibody. The term "antigen-binding fragment" of an antibody (or simply "antibody portion," or "fragment"), as used herein, may be defined as follows:
(1) Fab, the fragment which contains a monovalent antigen-binding fragment of an antibody molecule, can be produced by digestion of whole antibody with the enzyme papain to yield an intact light chain and a portion of one heavy chain;
(2) Fab', the fragment of an antibody molecule that can be obtained by treating whole antibody with pepsin, followed by reduction, to yield an intact light chain and a portion of the heavy chain; two Fab' fragments are obtained per antibody molecule;
(3) (Fab')2, the fragment of the antibody that can be obtained by treating whole antibody with the enzyme pepsin without subsequent reduction; F(ab')2 is a dimer of two Fab' fragments held together by two disulfide bonds;
(4) Fv, defined as a genetically engineered fragment containing the variable region of the light chain and the variable region of the heavy chain expressed as two chains; and
(5) Single chain antibody ("SCA", or ScFv), a genetically engineered molecule containing the variable region of the light chain and the variable region of the heavy chain, linked by a suitable polypeptide linker as a genetically fused single chain molecule.

Methods of generating such antibody fragments are well known in the art (See for example, Harlow and Lane, Antibodies: A Laboratory Manual, Cold Spring Harbor Laboratory, New York, 1988, incorporated herein by reference).

Purification of serum immunoglobulin antibodies (polyclonal antisera) or reactive portions thereof can be accomplished by a variety of methods known to those of skill in the art including, precipitation by ammonium sulfate or sodium sulfate followed by dialysis against saline, ion exchange chromatography, affinity or immuno-affinity chromatography as well as gel filtration, zone electrophoresis, etc.

Still further, for diagnostic and monitoring uses described herein after, the anti-SNX9 antibodies used by the present invention may optionally be covalently or non-covalently linked to a detectable label. The term "labeled" can refer to direct labeling of the antibody via, e.g., coupling (i.e., physically linking) a detectable substance to the antibody, and can also refer to indirect labeling of the antibody by reactivity with another reagent that is directly labeled. Examples of indirect labeling include detection of a primary antibody using a fluorescently labeled secondary antibody. More specifically, detectable labels suitable for such use include any composition detectable by spectroscopic, photochemical, biochemical, immunochemical, electrical, optical or chemical means. Useful labels in the present invention include magnetic beads (e.g. DYNABEADS), fluorescent dyes (e.g., fluorescein isothiocyanate, Texas red, rhodamine, green fluorescent protein, and the like), radiolabels (e.g., ³H, ¹²⁵I, ³⁵S, ¹⁴C, or ³²P), enzymes (e.g., horseradish peroxidase, alkaline phosphatase and others commonly used in an ELISA and competitive ELISA and other similar methods known in the art) and colorimetric labels such as colloidal gold or colored glass or plastic (e.g. polystyrene, polypropylene, latex, etc.) beads.

Means of detecting such labels are well known to those of skill in the art. Thus, for example, radiolabels may be detected using photographic film or scintillation counters, fluorescent markers may be detected using a photodetector to detect emitted illumination. Enzymatic labels are typically detected by providing the enzyme with a substrate and detecting the reaction product produced by the action of the enzyme on the substrate, and colorimetric labels are detected by simply visualizing the colored label.

The antibody used as a detecting molecule according to the invention, specifically recognizes and binds SNX9. It should be therefore noted that the term "binding specificity", "specifically binds to an antigen", "specifically immuno-reactive with", "specifically directed against" or "specifically recognizes", when referring to an epitope, specifically, a recognized epitope within the SNX9 molecule, refers to a binding reaction which is determinative of the presence of the epitope in a heterogeneous population of proteins and other biologics. More particularly, "selectively bind" in the context of proteins encompassed by the invention refers to the specific interaction of a any two of a peptide, a protein, a polypeptide an antibody, wherein the interaction preferentially occurs as between any two of a peptide, protein, polypeptide and antibody preferentially as compared with any other peptide, protein, polypeptide and antibody.

Thus, under designated immunoassay conditions, the specified antibodies bind to a particular epitope at least two times the background and more typically more than 10 to 100 times background. More specifically, "Selective binding", as the term is used herein, means that a molecule binds its specific binding partner with at least 2-fold greater affinity, and preferably at least 10-fold, 20-fold, 50-fold, 100-fold or higher affinity than it binds a non- specific molecule.

A variety of immunoassay formats may be used to select antibodies specifically immunoreactive with a particular protein or carbohydrate. For example, solid-phase ELISA immunoassays are routinely used to select antibodies specifically immunoreactive with a protein or carbohydrate. The term "epitope" is meant to refer to that portion of any molecule capable of being bound by an antibody which can also be recognized by that antibody. Epitopes or "antigenic determinants" usually consist of chemically active surface groupings of molecules such as amino acids or sugar side chains and have specific three dimensional structural characteristics as well as specific charge characteristics.

According to one embodiment, where amino acid-based detection molecules are used, the expression level of the SNX9 protein, in the tested sample can be determined using different methods known in the art, specifically method disclosed herein below as non-limiting examples.

### Enzyme-Linked Immunosorbent Assay (ELISA)

This method involves fixation of a sample containing a protein substrate (e.g., fixed cells or a proteinaceous solution) to a surface such as a well of a microtiter plate. A substrate-specific antibody coupled to an enzyme is applied and allowed to bind to the substrate. Presence of the antibody is then detected and quantitated by a colorimetric reaction employing the enzyme coupled to the antibody. Enzymes commonly employed in this method include horseradish peroxidase and alkaline phosphatase. If well calibrated and within the linear range of response, the amount of substrate present in the sample is proportional to the amount of color produced. A substrate standard is generally employed to improve quantitative accuracy.

### Western Blot

This method involves separation of a substrate from other protein by means of an acrylamide gel followed by transfer of the substrate to a membrane (e.g., nitrocellulose, nylon, or PVDF). Presence of the substrate is then detected by antibodies specific to the substrate, which are in turn detected by antibody -binding reagents. Antibody -binding reagents may be, for example, protein A or secondary antibodies. Antibody -binding reagents may be radiolabeled or enzyme-linked, as described hereinafter. Detection may be by autoradiography, colorimetric reaction, or chemiluminescence. This method allows both quantitation of an amount of substrate and determination of its identity by a relative position on the membrane indicative of the protein's migration distance in the acrylamide gel during electrophoresis, resulting from the size and other characteristics of the protein.

### Radioimmunoassay (RIA)

In one version, this method involves precipitation of the desired protein (i.e., the substrate) with a specific antibody and radiolabeled antibody -binding protein (e.g., protein A labeled with I¹²⁵) immobilized on a precipitable carrier such as agarose beads. The radio-signal detected in the precipitated pellet is proportional to the amount of substrate bound.

In an alternate version of RIA, a labeled substrate and an unlabelled antibody-binding protein are employed. A sample containing an unknown amount of substrate is added in varying amounts. The number of radio counts from the labeled substrate-bound precipitated pellet is proportional to the amount of substrate in the added sample.

### Fluorescence-Activated Cell Sorting (FACS)

This method involves detection of a substrate in situ in cells bound by substrate-specific, fluorescently labeled antibodies. The substrate-specific antibodies are linked to fluorophores. Detection is by means of a flow cytometry machine, which reads the wavelength of light emitted from each cell as it passes through a light beam. This method may employ two or more antibodies simultaneously, and is a reliable and reproducible procedure used by the present invention.

### Immunohistochemical Analysis

This method involves detection of a substrate in situ in fixed cells by substrate-specific antibodies. The substrate specific antibodies may be enzyme-linked or linked to fluorophores. Detection is by microscopy, and is either subjective or by automatic evaluation. With enzyme-linked antibodies, a calorimetric reaction may be required. It will be appreciated that immunohistochemistry is often followed by counterstaining of the cell nuclei, using, for example, Hematoxyline or Giemsa stain.

According to certain alternative embodiments, the detecting molecules for SNX9 expression may be isolated detecting nucleic acid molecules. According to some embodiments, such detecting nucleic acid molecules may be isolated oligonucleotides, each oligonucleotide specifically hybridizes to a nucleic acid sequence of the RNA products of said SNX9. More specifically, the oligonucleotide used as a detecting molecule according to certain embodiments of the invention may be any one of a pair of primers or nucleotide probe. In such case, the level of expression of SNX9 may be determined using a nucleic acid amplification assay selected from the group consisting of: a Real-Time PCR, micro arrays, PCR, in situ Hybridization and Comparative Genomic Hybridization. It should be noted that in particular embodiments, the invention further encompasses the use of aptamers as a nucleic acid based detection molecules that specifically recognize and bind the SNX9 protein.

As used herein, "nucleic acid(s)" is interchangeable with the term "polynucleotide(s)" and it generally refers to any polyribonucleotide or poly-deoxyribonucleotide, which may be unmodified RNA or DNA or modified RNA or DNA or any combination thereof. "Nucleic acids" include, without limitation, single- and double-stranded nucleic acids. As used herein, the term "nucleic acid(s)" also includes DNAs or RNAs as described above that contain one or more modified bases. Thus, DNAs or RNAs with backbones modified for stability or for other reasons are "nucleic acids". The term "nucleic acids" as it is used herein embraces such chemically, enzymatically or metabolically modified forms of nucleic acids, as well as the chemical forms of DNA and RNA characteristic of viruses and cells, including for example, simple and complex cells. A "nucleic acid" or "nucleic acid sequence" may also include regions of single- or double- stranded RNA or DNA or any combinations.

As used herein, the term "oligonucleotide" is defined as a molecule comprised of two or more deoxyribonucleotides and/or ribonucleotides, and preferably more than three. Its exact size will depend upon many factors which in turn, depend upon the ultimate function and use of the oligonucleotide. The oligonucleotides may be from about 8 to about 1,000 nucleotides long. Although oligonucleotides of 5 to 100 nucleotides are useful in the invention, preferred oligonucleotides range from about 5 to about 15 bases in length, from about 5 to about 20 bases in length, from about 5 to about 25 bases in length, from about 5 to about 30 bases in length, from about 5 to about 40 bases in length or from about 5 to about 50 bases in length. More specifically, the detecting oligonucleotides molecule used by the composition of the invention may comprise any one of 5, 6, 7, 8, 9, 10, 11, 12, 13, 14, 15, 16, 17, 18, 19, 20, 21, 22, 23, 24, 25, 26, 27, 28, 29, 30, 35, 40, 45, 50 bases in length. It should be further noted that the term "oligonucleotide" refers to a single stranded or double stranded oligomer or polymer of ribonucleic acid (RNA) or deoxyribonucleic acid (DNA) or mimetics thereof. This term includes oligonucleotides composed of naturally-occurring bases, sugars and covalent internucleoside linkages (e.g., backbone) as well as oligonucleotides having non-naturally-occurring portions which function similarly.

According to another alternative embodiment, where nucleic acid-based detection molecules are used, the expression level of the SNX9 RNA product, in the tested sample can be determined using methods known in the art. The following summarizes methods of determining levels of the expression of the biomarker of the invention, SNX9 (i.e., RNA or protein) in biological samples using nucleic acid base detection methods.

### Northern Blot Analysis

This method involves the detection of a particular RNA in a mixture of RNAs. An RNA sample is denatured by treatment with an agent (e.g., formaldehyde) that prevents hydrogen bonding between base pairs, ensuring that all the RNA molecules have an unfolded, linear conformation. The individual RNA molecules are then separated according to size by gel electrophoresis and transferred to a nitrocellulose or a nylon-based membrane to which the denatured RNAs adhere. The membrane is then exposed to labeled DNA probes. Probes may be labeled using radioisotopes or enzyme-linked nucleotides. Detection may be performed by autoradiography, colorimetric reaction, or chemiluminescence. This method allows for both quantitation of an amount of a particular RNA molecule and determination of its identity by a relative position on the membrane which is indicative of a migration distance in the gel during electrophoresis.

Polymerase chain reaction (PCR)-based methods (e.g., RT-PCR) may also be used to identify SNX9. For PCR-based methods a pair of oligonucleotides as the detection molecule is used, which is specifically hybridizable with the SNX9 polynucleotide sequences in an opposite orientation so as to direct exponential amplification of a portion thereof in a nucleic acid amplification reaction. The polymerase chain reaction and other nucleic acid amplification reactions are well known in the art and require no further description herein. The pair of oligonucleotides according to this aspect of the present invention are preferably selected to have compatible melting temperatures (Tm), e.g., melting temperatures which differ by less than that 7° C., preferably less than 5° C., more preferably less than 4° C., most preferably less than 3° C., ideally between 3° C. and 0° C.

### RT-PCR Analysis

This method uses PCR amplification of relatively rare RNA molecules. First, RNA molecules are purified from cells and converted into complementary DNA (cDNA) using a reverse transcriptase enzyme (such as an MMLV-RT) and primers such as oligo-dT, random hexamers, or gene-specific primers. Then by applying gene-specific primers and Taq DNA polymerase, a PCR amplification reaction is carried out in a PCR machine. Those of ordinary skill in the art are capable of selecting the length and sequence of the gene-specific primers and the PCR conditions (i.e., annealing temperatures, number of cycles, and the like) that are suitable for detecting specific RNA molecules. It will be appreciated that a semi-quantitative RT-PCR reaction can be employed, by adjusting the number of PCR cycles and comparing the amplification product to known controls. An example for Real-time PCR used in the method of the invention is presented by Figure 6E.

### RNA In Situ Hybridization Stain

In this method DNA or RNA probes are attached to the RNA molecules present in the cells. Generally, the cells are first fixed to microscopic slides to preserve the cellular structure and to prevent the RNA molecules from being degraded, and then are subjected to hybridization buffer containing the labeled probe. The hybridization buffer includes reagents such as formamide and salts (e.g., sodium chloride and sodium citrate) which enable specific hybridization of the DNA or RNA probes with their target mRNA molecules in situ while avoiding non-specific binding of probe. Those skilled in the art are capable of adjusting hybridization conditions (i.e., temperature, concentration of salts and formamide, and the like) for specific probes and types of cells. Following hybridization, any unbound probe is washed off and the slide is subjected to either a photographic emulsion, which reveals signals generated using radio-labeled probes, or to a calorimetric reaction, which reveals signals generated using enzyme-linked labeled probes.

### In Situ RT-PCR Stain

The RT-PCR reaction on fixed cells involves the incorporation of labeled nucleotides in the reaction. The reaction is effected using a specific in situ RT-PCR apparatus, such as the laser-capture microdissection PixCell II™ Laser Capture Microdissection (LCM) system available from Arcturus Engineering (Mountainview, Calif., USA).

### Oligonucleotide Microarray

In this method, oligonucleotide probes capable of specifically hybridizing with the polynucleotides of the present invention are attached to a solid surface (e.g., a glass wafer). Each oligonucleotide probe is of approximately 20-25 nucleic acids in length. To detect the expression pattern of the polynucleotides of the present invention in a specific cell sample (e.g., blood cells), RNA is extracted from the cell sample using methods known in the art (using, e.g., a TRIZOL® solution, Gibco-BRL™, USA). Hybridization can take place using either labeled oligonucleotide probes (e.g., 5'-biotinylated probes) or labeled fragments of complementary DNA (cDNA) or RNA (cRNA). Briefly, double-stranded cDNA is prepared from the RNA using reverse transcriptase (RT) (e.g., Superscript™ II RT), DNA ligase, and DNA polymerase I, all according to the manufacturer's instructions (Invitrogen Life Technologies, Frederick, Md., USA). To prepare labeled cRNA, the double-stranded cDNA is subjected to an in vitro transcription reaction in the presence of biotinylated nucleotides using, e.g., the BioArray™ HighYield™ RNA Transcript Labeling Kit (Enzo Diagnostics, Inc., Farmingdale, N.Y., USA). For efficient hybridization the labeled cRNA can be fragmented by incubating the RNA in 40 mM Tris Acetate (pH 8.1), 100 mM potassium acetate, and 30 mM magnesium acetate, for 35 minutes at 94° C. Following hybridization, the microarray is washed and the hybridization signal is scanned using a confocal laser fluorescence scanner, which measures fluorescence intensity emitted by the labeled cRNA bound to the probe arrays.

For example, in the Affymetrix® GeneChip® Microarray (Affymetrix, Inc., Santa Clara, Calif., USA), each gene on the array is represented by a series of different oligonucleotide probes, of which each probe pair consists of a perfect-match oligonucleotide and a mismatch oligonucleotide. While the perfect-match probe has a sequence exactly complimentary to the particular gene, thus enabling the measurement of the level of expression of the particular gene, the mismatch probe differs from the perfect match probe by a single base substitution at the center base position. The hybridization signal is scanned using the Agilent DNA Microarray Scanner™ (Agilent Technologies, USA) and the Microarray Suite™ (MAS) (Affymetrix, Inc.) software subtracts the non-specific signal of the mismatch probe from the signal resulting from the perfect-match probe.

It should be appreciated that all the detecting molecules (either nucleic acid based or amino acid based) used by the diagnostic methods and kits (kits will be described herein after) of the invention are isolated and/or purified molecules. As used herein, "isolated" or "purified" when used in reference to a nucleic acid means that a naturally occurring sequence has been removed from its normal cellular (e.g., chromosomal) environment or is synthesized in a non-natural environment (e.g., artificially synthesized). Thus, an "isolated" or "purified" sequence may be in a cell-free solution or placed in a different cellular environment. The term "purified" does not imply that the sequence is the only nucleotide present, but that it is essentially free (about 90-95% pure) of non- nucleotide material naturally associated with it, and thus is distinguished from isolated chromosomes. As used herein, the terms "isolated" and "purified" in the context of a proteinaceous agent (e.g., a peptide, polypeptide, protein or antibody) refer to a proteinaceous agent which is substantially free of cellular material and in some embodiments, substantially free of heterologous proteinaceous agents (i.e. contaminating proteins) from the cell or tissue source from which it is derived, or substantially free of chemical precursors or other chemicals when chemically synthesized. The language "substantially free of cellular material" includes preparations of a proteinaceous agent in which the proteinaceous agent is separated from cellular components of the cells from which it is isolated or recombinantly produced. Thus, a proteinaceous agent that is substantially free of cellular material includes preparations of a proteinaceous agent having less than about 30%, 20%, 10%, or 5% (by dry weight) of heterologous proteinaceous agent (e.g. protein, polypeptide, peptide, or antibody; also referred to as a "contaminating protein"). When the proteinaceous agent is recombinantly produced, it is also preferably substantially free of culture medium, i.e. culture medium represents less than about 20%, 10%, or 5% of the volume of the protein preparation. When the proteinaceous agent is produced by chemical synthesis, it is preferably substantially free of chemical precursors or other chemicals, i.e., it is separated from chemical precursors or other chemicals which are involved in the synthesis of the proteinaceous agent. Accordingly, such preparations of a proteinaceous agent have less than about 30%, 20%, 10%, 5% (by dry weight) of chemical precursors or compounds other than the proteinaceous agent of interest. Preferably, proteinaceous agents disclosed herein are isolated.

According to certain embodiments, the reference control protein used by the methods of the invention displays constant expression pattern in a healthy and a diseased sample. Such control may be for example, at least one of CD3ε, CD3δ, CD3γ, TCRα and TCRβ, for T cells, CD19 for B cells and SNX27 for all cells. As shown by the following Examples, the expression of CD3ε, CD19 and SNX27, is similar in samples displaying chronic inflammation and in non-inflammatory samples. Therefore, in a specific embodiment, CD3ε is used as a control reference for T cells, CD19 is used as a control reference for B cells and SNX27 is used as a reference control for all cells.

The present invention provides a powerful tool for determining the immune status of a subject, specifically, chronic inflammation and associated immune-suppression. However, in certain embodiments, it should be noted that the method of the invention may be specifically applicable for detecting and monitoring chronic inflammation in a subject suffering from a chronic inflammatory condition. More specifically, in some embodiments, the method of the invention may be applicable for detecting and monitoring chronic inflammation in a subject suffering from a chronic inflammatory condition. Such condition may be any one of an autoimmune disease, a proliferative disorder and an infectious disease.

According to one embodiment, the method of the invention may be used for detecting and monitoring chronic inflammation in a subject suffering from an autoimmune disease. In certain embodiments, such autoimmune disease may be an inflammatory arthritis.

As shown by Example 5, using the collagen induced arthritis model (CIA), the inventors demonstrated the feasibility of using SNX9 as a biomarker for detecting and monitoring the immune-status, specifically, chronic inflammation in chronic inflammatory conditions such as arthritis. Thus, according to specific embodiments, the expression levels of SNX9 are reduced or decreased in subjects suffering from arthritis. More specifically, the reduction observed may be of about at least a 1.5-fold decrease, at least a 2-fold decrease, at least a 3-fold decrease, at least a 4-fold decrease, at least a 5-fold decrease, at least a 6-fold decrease, at least a 7-fold decrease, at least a 8-fold decrease, at least a 9-fold decrease, or, at least 10-fold decrease or more decrease in SNX9 expression levels as compared to healthy control subjects or subjects that do not display chronic inflammation.

As indicated by Berent, J. et al., [Berent, J. et al., Springer Semin. Immunopathol. 25: 7-63 (2003)], adjuvant arthritis (AA) is a well established animal model for rheumatoid arthritis (RA), juvenile idiopathic arthritis (JIA) and septic arthritis. Moreover, different publications [Myers et al. Life Sciences 61(19): 1861-1878 (1997) and Brand et al. Springer Semin Immunopathol (2003) 25:3-18 (2003), respectively], clearly indicate that collagen induced arthritis (CIA) is also an established model for RA as well as to other autoimmunity, rheumatic diseases and inflammation.

It should be appreciated that there are different forms of arthritis that may be generally grouped into two main categories, inflammatory arthritis, and degenerative arthritis, each with different causes. Therefore, according to one specific embodiment, the diagnostic methods of the invention may be specifically intended for the detection and/or monitoring of chronic inflammation in subjects suffering from a chronic inflammatory condition, specifically, an autoimmune disease, for example, an inflammatory arthritis. Inflammatory arthritis is characterized by synovitis, bone erosions, osteopenia, soft-tissue swelling, and uniform joint space narrowing. It should be appreciated that inflammatory arthritis may be further divided into several subgroups, and therefore, the diagnostic methods, compositions and kits of the invention described herein after, may be applicable for detection and monitoring of chronic inflammation status in subjects suffering from every inflammatory arthritis condition of the different subgroups.

More specifically, involvement of a single joint is indicative of a Septic arthritis. The cause of septic arthritis is usually related to hematogenous seeding owing to staphylococcal or streptococcal microorganisms. According to one embodiment, the diagnostic methods, compositions and kits of the invention may be used for detection and monitoring of chronic inflammation status in subjects suffering from septic arthritis.

A systemic arthritis, in contrast, is characterized by involvement multiple joints, and includes two main categories, rheumatoid arthritis and seronegative spondyloarthropathy.

According to one embodiment, the diagnostic and prognostic methods, and kits of the invention may be used for detection and monitoring of chronic inflammation status in subjects suffering from rheumatoid arthritis. Rheumatoid arthritis (RA) is a chronic, systemic autoimmune disorder that most commonly causes inflammation and tissue damage in joints (arthritis) and tendon sheaths, together with anemia. It can also produce diffuse inflammation in the lungs, pericardium, pleura, and the sclera of the eye, and also nodular lesions, most common in subcutaneous tissue. It can be a disabling and painful condition, which can lead to substantial loss of functioning and mobility. Serologic markers such as rheumatoid factor and antibodies to cyclic citrullinated peptide are important indicators of rheumatoid arthritis. The radiographic features of rheumatoid arthritis are those of joint inflammation and include particular osteopenia, uniform joint space loss, bone erosions, and soft-tissue swelling. Because of the chronic nature of the inflammation, additional findings such as joint subluxation and subchondral cysts may also be evident.

The seronegative spondyloarthropathy category includes psoriatic arthritis, reactive arthritis, and ankylosing spondylitis, and is characterized by signs of inflammation, multiple joint involvement, and distal involvement in the hands and feet with added features of bone proliferation. Thus, according to one embodiment, the diagnostic and prognostic methods, compositions and kits of the invention may be used for detection and monitoring of chronic inflammation status in subjects suffering from any condition of the seronegative spondyloarthropathy category.

More specifically, the diagnostic and prognostic methods, and kits of the invention may be used for detection and monitoring of chronic inflammation status in subjects suffering from Psoriatic arthritis. This condition is a chronic disease characterized by inflammation of the skin (psoriasis) and joints (arthritis). In yet another embodiment, the diagnostic and prognostic methods, compositions and kits of the invention may be used for detection and monitoring of chronic inflammation status in subjects suffering from Psoriasis. One of the characteristics of psoriatic arthritis is a common skin condition that features patchy, raised, red areas of skin inflammation with scaling.

According to another embodiment, the diagnostic and prognostic methods, and kits of the invention may be used for detection and monitoring of chronic inflammation status in subjects suffering from ankylosing spondylitis. Ankylosing spondylitis (AS, previously known as Bechterew's disease, Bechterew syndrome, Marie Strümpell disease and a form of spondyloarthritis), is usually a chronic and progressive form of arthritis, caused due to inflammation of multiple joints, characteristically the spinal facet joints and the sacroiliac joints at the base of the spine.

In yet another embodiment, the diagnostic and prognostic methods, and kits of the invention may be used for detection and monitoring of chronic inflammation status in subjects suffering from reactive arthritis (ReA). Reactive arthritis, that is another type of seronegative spondyloarthropathy, is an autoimmune condition that develops in response to an infection in another part of the body. Coming into contact with bacteria and developing an infection can trigger reactive arthritis. It has symptoms similar to various other conditions collectively known as "arthritis," such as rheumatism. It is caused by another infection and is thus "reactive", i.e., dependent on the other condition. The "trigger" infection has often been cured or is in remission in chronic cases, thus making determination of the initial cause difficult.

It should be appreciated that there are many other forms of inflammatory arthritis, including juvenile idiopathic arthritis, gout and pseudo gout, as well as arthritis associated with colitis or psoriasis. It should be therefore appreciated that the diagnostic methods, and kits of the invention are also applicable for these conditions as well. Therefore, according to another embodiment, the diagnostic and prognostic methods, and kits of the invention may be used for detection and monitoring of chronic inflammation status in subjects suffering from juvenile idiopathic arthritis (JIA). JIA is the most common form of persistent arthritis in children. (juvenile in this context refers to an onset before age 16, idiopathic refers to a condition with no defined cause, and arthritis is the inflammation of the synovium of a joint). JIA is a subset of arthritis seen in childhood, which may be transient and self-limited or chronic. It differs significantly from arthritis commonly seen in adults (rheumatoid arthritis), and other types of arthritis that can present in childhood which are chronic conditions (e.g. psoriatic arthritis and ankylosing spondylitis).

Generally, as also disclosed above, there are many types of arthritis, it should be noted that in addition to all primary forms of arthritis indicated, the diagnostic methods, compositions and kits of the invention may be also applicable for detection and monitoring of chronic inflammation status in subjects suffering from all secondary forms of arthritis. These conditions may include lupus erythematosus, Henoch-Schönlein purpura, psoriatic arthritis, reactive arthritis, haemochromatosis, hepatitis, Wegener's granulomatosis (and many other vasculitis syndromes), Lyme disease, familial mediterranean fever, hyperimmunoglobulinemia D with recurrent fever, TNF receptor associated periodic syndrome and inflammatory bowel disease (including Crohn's Disease and ulcerative colitis).

Although exemplified for arthritis as a non-limiting example for an autoimmune disorder, it should be appreciated that the diagnostic and prognostic methods, compositions and kits of the invention may be also applicable for detection and monitoring of chronic inflammation status in subjects suffering from any other autoimmune disorder, for example, IBD. Inflammatory bowel diseases (IBD) are common gastrointestinal disorders, that can be perceived as being the result of a dysbalance between Th1-pro-inflammatory and Th2-anti-inflammatory subtypes of immune responses. IBD is a group of inflammatory conditions of the colon and small intestine. The major types of IBD are Crohn's disease and ulcerative colitis (UC) that share the same symptoms such as diarrhea, vomiting, weight loss, fever and abdominal pain. Other forms of IBD account for far fewer cases. These are Collagenous colitis, Lymphocytic colitis, Ischaemic colitis, Diversion colitis, Behçet's syndrome and Indeterminate colitis which is inability to make a definitive diagnosis distinguishing Crohn's disease from Ulcerative colitis.

In yet another specific embodiment for an autoimmune condition, the diagnostic and prognostic methods, and kits of the invention may be also applicable for detection and monitoring of chronic inflammation status in subjects suffering from diabetes.

Diabetes mellitus, is a syndrome characterized by disordered metabolism and inappropriately high blood sugar (hyperglycaemia) resulting from either low levels of the hormone insulin or from abnormal resistance to insulin's effects coupled with inadequate levels of insulin secretion to compensate. The characteristic symptoms are excessive urine production (polyuria), excessive thirst and increased fluid intake (polydipsia), and blurred vision; these symptoms are likely absent if the blood sugar is only mildly elevated.

There are three main forms of diabetes: type 1, type 2 and gestational diabetes (occurs during pregnancy). Type 1 diabetes mellitus is characterized by loss of the insulin-producing beta cells of the islets of Langerhans in the pancreas, leading to a deficiency of insulin. The main cause of this beta cell loss is a T-cell mediated autoimmune attack. There is no known preventative measure that can be taken against type 1 diabetes. Most affected people are otherwise healthy and of a healthy weight when onset occurs. Sensitivity and responsiveness to insulin are usually normal, especially in the early stages. Type 1 diabetes can affect children or adults and was traditionally termed "juvenile diabetes" as it represents a majority of cases of diabetes affecting children.

Diabetes mellitus type 2, or non-insulin-dependent diabetes mellitus (NIDDM) or adult-onset diabetes, is a metabolic disorder that is characterized by high blood glucose in the context of insulin resistance and relative insulin deficiency. As the condition progresses, medications may be needed. Long-term complications from high blood sugar include an increased risk of heart attacks, strokes, amputation, and kidney failure. There are many factors which can potentially give rise to or exacerbate type 2 diabetes. These include obesity, hypertension, elevated cholesterol (combined hyperlipidemia), and with the condition often termed metabolic syndrome (it is also known as Syndrome X, Reavan's syndrome, or CHAOS). Other causes include acromegaly, Cushing's syndrome, thyrotoxicosis, pheochromocytoma, chronic pancreatitis, cancer and drugs. Additional factors found to increase the risk of type 2 diabetes include aging, high-fat diets and a less active lifestyle.

There is growing evidence that there may be a link between inflammation and the pathogenesis of Type 2 diabetes. This evolving concept which suggests that insulin resistance and type 2 diabetes may have an immune component provides a new avenue to investigate immunotherapeutic approaches to both understand the pathogenesis of type 2 diabetes and to develop new treatments for the disease.

In yet another example for an autoimmune disorder, the diagnostic and prognostic methods, and kits of the invention may be used for detection and monitoring of chronic inflammation status in subjects suffering from Multiple sclerosis. More specifically, Multiple sclerosis (abbreviated MS, formerly known as disseminated sclerosis or encephalomyelitis disseminata) is a chronic, inflammatory, demyelinating disease that affects the central nervous system (CNS). Disease onset usually occurs in young adults, is more common in women, and has a prevalence that ranges between 2 and 150 per 100,000 depending on the country or specific population.

MS takes several forms, with new symptoms occurring either in discrete episodes (relapsing forms) or slowly accumulating over time (progressive forms). Most people are first diagnosed with relapsing-remitting MS but develop secondary-progressive MS (SPMS) after a number of years. Between episodes or attacks, symptoms may go away completely, but permanent neurological problems often persist, especially as the disease advances.

It should be further appreciated that in general, the diagnostic and prognostic methods, and kits of the invention may be also applicable for detection and monitoring of chronic inflammation status in subjects suffering from any autoimmune disease such as for example, but not limited to, Eaton-Lambert syndrome, Goodpasture's syndrome, Greave's disease, Guillain-Barr syndrome, autoimmune hemolytic anemia (AIHA), hepatitis, insulin-dependent diabetes mellitus (IDDM) and NIDDM, systemic lupus erythematosus (SLE), multiple sclerosis (MS), myasthenia gravis, plexus disorders e.g. acute brachial neuritis, polyglandular deficiency syndrome, primary biliary cirrhosis, rheumatoid arthritis, scleroderma, thrombocytopenia, thyroiditis e.g. Hashimoto's disease, Sjogren's syndrome, allergic purpura, psoriasis, mixed connective tissue disease, polymyositis, dermatomyositis, vasculitis, polyarteritis nodosa, arthritis, alopecia areata, polymyalgia rheumatica, Wegener's granulomatosis, Reiter's syndrome, Behget's syndrome, ankylosing spondylitis, pemphigus, bullous pemphigoid, dermatitis herpetiformis, inflammatory bowel disease, ulcerative colitis and Crohn's disease and fatty liver disease.

In yet another embodiment, the chronic inflammatory condition may be a proliferative disorder, for example, any one of melanoma, carcinoma sarcoma, glioma, leukemia and lymphoma. Thus, according to certain embodiments, the diagnostic methods and kits of the invention may be applicable for determining the immune-status, and specifically, a chronic inflammation, in a subject suffering from a proliferative disorder.

As used herein to describe the present invention, "proliferative disorder", "cancer", "tumor" and "malignancy" all relate equivalently to a hyperplasia of a tissue or organ. If the tissue is a part of the lymphatic or immune systems, malignant cells may include non-solid tumors of circulating cells. Malignancies of other tissues or organs may produce solid tumors. In general, the methods of the present invention may be applicable for determining the immune-status in patient suffering from any one of non-solid and solid tumors.

Malignancy, as contemplated in the present invention may be any one of melanomas, carcinomas, lymphomas, leukemias, myeloma and sarcomas.

Melanoma as used herein and will be described in more detail hereinafter, is a malignant tumor of melanocytes. Melanocytes are cells that produce the dark pigment, melanin, which is responsible for the color of skin. They predominantly occur in skin, but are also found in other parts of the body, including the bowel and the eye. Melanoma can occur in any part of the body that contains melanocytes.

Carcinoma as used herein, refers to an invasive malignant tumor consisting of transformed epithelial cells. Alternatively, it refers to a malignant tumor composed of transformed cells of unknown histogenesis, but which possess specific molecular or histological characteristics that are associated with epithelial cells, such as the production of cytokeratins or intercellular bridges.

Leukemia refers to progressive, malignant diseases of the blood-forming organs and is generally characterized by a distorted proliferation and development of leukocytes and their precursors in the blood and bone marrow. Leukemia is generally clinically classified on the basis of (1) the duration and character of the disease-acute or chronic; (2) the type of cell involved; myeloid (myelogenous), lymphoid (lymphogenous), or monocytic; and (3) the increase or non-increase in the number of abnormal cells in the blood-leukemic or aleukemic (subleukemic). Leukemia as used herein includes, for example, acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocyte leukemia, micromyeloblastic leukemia, monocytic leukemia, myeloblasts leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

Sarcoma is a cancer that arises from transformed connective tissue cells. These cells originate from embryonic mesoderm, or middle layer, which forms the bone, cartilage, and fat tissues. This is in contrast to carcinomas, which originate in the epithelium. The epithelium lines the surface of structures throughout the body, and is the origin of cancers in the breast, colon, pancreas.

Myeloma as mentioned herein, is a cancer of plasma cells, a type of white blood cell normally responsible for the production of antibodies. Collections of abnormal cells accumulate in bones, where they cause bone lesions, and in the bone marrow where they interfere with the production of normal blood cells. Most cases of myeloma also feature the production of a paraprotein, an abnormal antibody that can cause kidney problems and interferes with the production of normal antibodies leading to immunodeficiency. Hypercalcemia (high calcium levels) is often encountered.

Lymphoma is a cancer in the lymphatic cells of the immune system. Typically, lymphomas present as a solid tumor of lymphoid cells. These malignant cells often originate in lymph nodes, presenting as an enlargement of the node (a tumor). It can also affect other organs in which case it is referred to as extranodal lymphoma.

Further malignancies that may find utility in the present invention can comprise but are not limited to hematological malignancies (including lymphoma, leukemia and myeloproliferative disorders), hypoplastic and aplastic anemia (both virally induced and idiopathic), myelodysplastic syndromes, all types of paraneoplastic syndromes (both immune mediated and idiopathic) and solid tumors (including GI tract, colon, lung, liver, breast, prostate, pancreas and Kaposi's sarcoma. More particularly, the malignant disorder may be lymphoma. Non-limiting examples of cancers treatable according to the invention include hematopoietic malignancies such as all types of lymphomas, leukemia, e.g. acute lymphocytic leukemia (ALL), acute myelogenous leukemia (AML), chronic lymphocytic leukemia (CLL), chronic myelogenous leukemia (CML), myelodysplastic syndrome (MDS), mast cell leukemia, hairy cell leukemia, Hodgkin's disease, non-Hodgkin's lymphomas, Burkitt's lymphoma and multiple myeloma, as well as for the treatment or inhibition of solid tumors such as tumors in lip and oral cavity, pharynx, larynx, paranasal sinuses, major salivary glands, thyroid gland, esophagus, stomach, small intestine, colon, colorectum, anal canal, liver, gallbladder, extraliepatic bile ducts, ampulla of vater, exocrine pancreas, lung, pleural mesothelioma, bone, soft tissue sarcoma, carcinoma and malignant melanoma of the skin, breast, vulva, vagina, cervix uteri, corpus uteri, ovary, fallopian tube, gestational trophoblastic tumors, penis, prostate, testis, kidney, renal pelvis, ureter, urinary bladder, urethra, carcinoma of the eyelid, carcinoma of the conjunctiva, malignant melanoma of the conjunctiva, malignant melanoma of the uvea, retinoblastoma, carcinoma of the lacrimal gland, sarcoma of the orbit, brain, spinal cord, vascular system, hemangiosarcoma and Kaposi's sarcoma.

Example 6 demonstrates the feasibility of using SNX9 as a biomarker for chronic inflammation in melanoma patients. Thus, in one specific embodiment, the diagnostic and prognostic method of the invention may be used for determining the immune-status of a patient suffering from melanoma. The term melanoma includes, but is not limited to, melanoma, metastatic melanoma, melanoma derived from either melanocytes or melanocyte-related nevus cells, melanocarcinoma, melanoepithelioma, melanosarcoma, melanoma *in situ,* superficial spreading melanoma, nodular melanoma, lentigo maligna melanoma, acral lentiginoous melanoma, invasive melanoma or familial atypical mole and melanoma (FAM-M) syndrome. Such melanomas may be caused by chromosomal abnormalities, degenerative growth and developmental disorders, mitogenic agents, ultraviolet radiation (UV), viral infections, inappropriate tissue gene expression, alterations in gene expression, or carcinogenic agents.

The applicability of diagnostic and prognostic method of the invention in detecting chronic inflammation in carcinomas, such as colorectal carcinoma, is demonstrated by Example 7. Therefore, in yet another specific embodiment, the diagnostic method of the invention may be used for determining the immune-status of a patient suffering from colorectal carcinoma.

In yet another embodiment, the chronic inflammatory condition may be an infectious disease. More specifically, such infectious disease may be any one of protozoan diseases, viral diseases, bacterial diseases, parasitic diseases, fungal diseases and mycoplasma diseases. As shown by Example 8, decreased expression of SNX9 in subjects infected with *Leishmania donovani,* indicates a chronic inflammation in the tested subject. Therefore, specific embodiments of the invention relate to the use of the method of the invention for detecting and monitoring chronic inflammation in a subject suffering from a protozoan diseases, specifically, a subject infected with *Leishmania.*

It should be appreciated that an infectious disease as used herein also encompasses any infectious disease caused by a pathogenic agent. Pathogenic agents include prokaryotic microorganisms, lower eukaryotic microorganisms, complex eukaryotic organisms, viruses, fungi, prions, parasites, yeasts, toxins and venoms.

A prokaryotic microorganism includes bacteria such as Gram positive, Gram negative and Gram variable bacteria and intracellular bacteria. Examples of bacteria contemplated herein include the species of the genera *Treponema sp., Borrelia sp., Neisseria sp., Legionella sp., Bordetella sp., Escherichia sp., Salmonella sp., Shigella sp., Klebsiella sp., Yersinia sp., Vibrio sp., Hemophilus sp., Rickettsia sp., Chlamydia sp., Mycoplasma sp., Staphylococcus sp., Streptococcus sp., Bacillus sp., Clostridium sp., Corynebacterium sp., Proprionibacterium sp., Mycobacterium sp., Ureaplasma sp. and Listeria sp.*

Particular species include *Treponema pallidum, Borrelia burgdorferi, Neisseria gonorrhea, Neisseria meningitidis, Legionella pneumophila, Bordetella pertussis, Escherichia coli, Salmonella typhi, Salmonella typhimurium, Shigella dysenteriae, Klebsiella pneumoniae, Yersinia pestis, Vibrio cholerae, Hemophilus influenzae, Rickettsia rickettsii, Chlamydia trachomatis, Mycoplasma pneumoniae, Staphylococcus aureus, Streptococcus pneumoniae, Streptococcus pyogenes, Bacillus anthracis, Clostridium botulinum, Clostridium tetani, Clostridium perfringens, Corynebacterium diphtheriae, Proprionibacterium acnes, Mycobacterium tuberculosis, Mycobacterium leprae* and *Listeria monocytogenes.*

A lower eukaryotic organism includes a yeast or fungus such as but not limited to *Pneumocystis carinii, Candida albicans,* Aspergillus, *Histoplasma capsulatum, Blastomyces dermatitidis, Cryptococcus neoformans,* Trichophyton and Microsporum.

A complex eukaryotic organism includes worms, insects, arachnids, nematodes, aemobe, *Entamoeba histolytica, Giardia lamblia, Trichomonas vaginalis, Trypanosoma brucei gambiense, Trypanosoma cruzi, Balantidium coli, Toxoplasma gondii,* Cryptosporidium or Leishmania.

The term "viruses" is used in its broadest sense to include viruses of the families adenoviruses, papovaviruses, herpesviruses: simplex, varicella-zoster, Epstein-Barr, CMV, pox viruses: smallpox, vaccinia, hepatitis B, rhinoviruses, hepatitis A, poliovirus, rubella virus, hepatitis C, arboviruses, rabies virus, influenza viruses A and B, measles virus, mumps virus, HIV, HTLV I and II.

The term "fungi" includes for example, fungi that cause diseases such as ringworm, histoplasmosis, blastomycosis, aspergillosis, cryptococcosis, sporotrichosis, coccidioidomycosis, paracoccidio-idoinycosis, and candidiasis.

The term parasite includes, but not limited to, infections caused by somatic tapeworms, blood flukes, tissue roundworms, ameba, and Plasmodium, Trypanosoma, Leishmania, and Toxoplasma species.

It should be noted that certain embodiments of the invention contemplate the use of different biological samples. The term "sample" in the present specification and claims is meant to include biological samples. Biological samples may be obtained from mammal, specifically, a human subject, include fluid, solid (e.g., stool) or tissues. The term "sample" may also include body fluids such as whole blood sample, blood cells, bone marrow, lymph fluid, serum, plasma, urine, sputum, saliva, faeces, semen, spinal fluid or CSF, the external secretions of the skin, respiratory, intestinal, and genitourinary tracts, tears, milk, any human organ or tissue, any biopsy, for example, lymph node or spleen biopsies, any sample taken from any tissue or tissue extract, any sample obtained by lavage optionally of the breast ductal system, plural effusion, samples of in vitro or ex vivo cell culture and cell culture constituents. Some samples that are a priori not liquid are contacted with a liquid buffers which are then used according to the diagnostic method of the invention.

Biological samples may be obtained from all of the various families of domestic animals, as well as feral or wild animals, including, but not limited to, such animals as ungulates, bear, fish, lagamorphs, rodents, etc. Preferably, the sample is liquid, specifically, a body fluid sample, most preferably, a serum sample and of mammalian origin, specifically, human. In specific embodiment, a blood sample is being used by the invention. In yet another specific embodiment, the method of the invention is applicable for testing any tissue sample, for example, lymph nodes and spleen, biopsies.

It should be noted that some embodiments of the invention encompass the use of the diagnostic method described herein for the diagnosis, prognosis, evaluating the immune status, and monitoring the effect of therapy in subjects suffering of a chronic inflammatory condition. More specifically, the method of the present invention provides a tool for determining whether to give immune based therapy. Still further, the prognostic method of the invention provides a sensitive tool for early detection of disease regression and reoccurrence.

Therefore, a second aspect of the invention relates to a method for evaluating the efficacy of a treatment with a therapeutic agent, given to a subject suffering from a chronic-inflammatory condition. It should be noted that the therapeutic agent may have a direct or indirect anti-inflammatory effect. The method comprises the step of:
The first step (a), involves determining the level of expression of SNX9 in a biological sample of the subject, to obtain SNX9 expression value in the tested sample. It should be noted that the sample should be obtained prior to initiation of said treatment. The second step (b) determining the level of expression of SNX9 in at least one other biological sample of the subject, to obtain SNX9 expression value in said sample. It should be noted that the at least one other sample is obtained after initiation of said treatment. In the third step (c), comparison of SNX9 expression value in the biological sample obtained in step (a), with at least one SNX9 expression value obtained in step (b), is required.

In certain embodiments, a higher SNX9 expression value in a sample obtained after initiation of said treatment according to step (b) as compared to the SNX9 expression value in a sample obtained prior to initiation of said treatment according to step (a), is indicative of successful therapy.

Example 9 demonstrates the feasibility of using the method of the invention in evaluating responsiveness to a successful treatment with a therapeutic agent such as 5FU, as an example for chemotherapy.

As used herein the phrase "predicting or evaluating efficacy of a treatment" refers to determining the likelihood that a specific treatment using a therapeutic agent is efficient or non-efficient in treating the chronic inflammatory condition, e.g., the success or failure of the treatment in treating the chronic inflammatory condition in a subject in need thereof. More specifically, a treatment with a therapeutic agent that directly, as an anti-inflammatory agent, or indirectly (a chemotherapeutic agent) effects inflammation. The term "efficacy" as used herein refers to the extent to which the anti-inflammatory treatment produces a beneficial result, e.g., an improvement in one or more symptoms of the pathology (caused by the chronic inflammatory condition) and/or clinical parameters related to the pathology as described hereinbelow. For example, the efficacy of an anti-inflammatory treatment may be evaluated using standard therapeutic indices for chronic inflammatory condition, for example, a proliferative disorder, an autoimmune disease or an infectious disease).

According to some embodiments of the invention, the efficacy of treatment is a long-term efficacy. As used herein the phrase "long-term efficacy" refers to the ability of a treatment to maintain a beneficial result over a period of time, e.g., at least about 16 weeks, at least about 26 weeks, at least about 32 weeks, at least about 36 weeks, at least about 40 weeks, at least about 48 weeks, at least about 52 weeks, at least about 18 months, at least about 24 months, at least about 3 years, at least about 4 years, at least about 5 years, at least about 6 years, at least about 7 years, at least about 8 years, at least about 9 years, at least about 10 years, or longer.

According to some embodiments of the invention, a treatment with a therapeutic agent that either directly or indirectly affects inflammation, is considered efficient in treating a chronic inflammatory condition if it exerts an improvement in at least one relevant clinical parameter related to said condition in the treated subject as compared to an untreated subject diagnosed with the same condition (e.g., where the chronic inflammatory condition is cancer, such parameter include the type, stage, degree and/or classification of the solid tumor), or as compared to the clinical parameters related to the said condition of the same subject prior to the anti-inflammatory treatment.

In certain embodiments the invention provides a method for monitoring and assessing responsiveness of a subject suffering from a chronic-inflammatory condition to a treatment with a therapeutic agent. It should be noted that the therapeutic agent leads to either a direct or indirect anti-inflammatory effect. The method comprising the steps of: The first step (a) involves contacting detecting molecules specific for SNX9 with a biological sample (or with an aliquot thereof) of said subject, or alternatively, with any protein or nucleic acid product obtained from the sample. It should be noted that optionally, the detecting molecules may be contacted with a control sample or with any protein or nucleic acid product obtained therefrom.

In a second step (b), contacting detecting molecules specific for at least one reference control, with a biological sample of said subject (or with an aliquot thereof), or with any protein or nucleic acid product obtained therefrom. Optionally, the control reference detecting molecules are contacted with a control sample or with any protein or nucleic acid product obtained therefrom.

A third step of the method (c) requires determining the expression value of SNX9 in the biological sample according to step (a), and optionally, in a suitable control sample.

The forth step (d), requires determining the expression value of said at least one reference control in the biological sample according to step (b), and optionally, in a suitable control sample.

The next step (e) involves comparing the SNX9 expression value in the test biological sample obtained in step (e), with a predetermined standard expression value, or a cutoff value, or with an expression value of SNX9 in a control sample optionally obtained in step (e).

In a further step (f) repeating steps (a) to (e) to obtain expression values of said SNX9, for at least one more temporally-separated test sample. It should be noted that a first sample is obtained prior to initiation of the treatment, and at least one more temporally-separated test sample is obtained after the initiation of said treatment.

Finally, in step (g), calculating the rate of change of said expression values of said SNX9 between said temporally-separated test samples.

It should be noted that a positive rate of change of said expression values in a sample obtained after initiation of the treatment as compared to the SNX9 expression value in a sample obtained prior to initiation of said treatment, is indicative of the responsiveness of said subject to said anti-inflammatory treatment.

In certain embodiments, the method of the invention further comprising an additional step of normalization of the expression values obtained in step (c) for the SNX9, according to the expression values of the control reference obtained in step (d), in said test sample. Optionally, similar normalization is performed also relative to a control sample when applicable.

In practice, for monitoring purpose, to detect a decline or elevation in SNX9 expression, at least two "temporally-separated" test samples must be collected from the treated patient, and preferably more. The expression of at least SNX9 is then determined using the method of the invention, applied for each sample. The rate of change in this biomarker expression is then calculated by determining the difference in normalized expression values of said SNX9 between any two samples and dividing the difference by the period of time that had over-lapsed between the collections of said at least two samples that are "temporally-separated" i.e., obtained from the same patient in different time-points or time intervals. This period of time, also referred to as "time interval", or the difference between time points (wherein each time point is the time when a specific sample was collected) may be any period deemed appropriate by medical staff and modified as needed according to the specific requirements of the patient and the clinical state he or she may be in. For example, this interval may be at least one day, at least three days, at least three days, at least one week, at least two weeks, at least three weeks, at least one month, at least two months, at least three months, at least four months, at least five months, at least one year, or even more.

When calculating the rate of change, one may use any two samples collected at different time points from the patient. To ensure more reliable results and reduce statistical deviations to a minimum, averaging the calculated rates of several sample pairs is preferable. A calculated or average negative rate of change of the normalized expression values of SNX9 indicates that the subject is in a chronic inflammation state.

As indicated above, in order to execute the diagnostic method of the invention, at least two different samples, and preferably, more than two, must be obtained, from the subject in order to calculate the rate of expression change in said SNX9. By obtaining at least two and preferably more biological samples from a subject and analyzing them according to the method of the invention, the diagnostic method may be effective for predicting, monitoring and early diagnosing molecular alterations indicating a chronic inflammation in said patient. Thus, the prognostic method of the invention may be applicable for early, sub-symptomatic diagnosis of chronic inflammation when used for analysis of more than a single sample along the time-course of diagnosis, treatment and follow-up. An "early diagnosis" provides diagnosis prior to appearance of clinical symptoms. Prior as used herein is meant days, weeks, months or even years before the appearance of such symptoms. More specifically, at least 1 week, at least 1 month, 2 months, 3 months, 4 months, 5 months, 6 months, 7 months, 8 months, 9 months, 10 months, 11 months, 12 months, or even few years before clinical symptoms appear.

Simply put, a decline in the expression of SNX9 indicates a chronic inflammation, and may provide an early sign before over symptoms occur, allowing for a quicker and more efficient therapeutic response.

More specifically, as indicated above, the invention relates to a prognostic method. Prognosis is defined as a forecast of the future course of a disease or disorder, based on medical knowledge. This highlights the major advantage of the instant invention over prior art, namely, the ability to predict chronic inflammation indicating regression or recurrence of the diseases in patients. This early prognosis facilitates the selection of appropriate treatment regimens that may minimize the predicted regression or recurrence of the diseases, individually to each patient, as part of personalized medicine.

It is therefore appreciated that the reduction in the expression of SNX9 constitutes an early marker of regression or recurrence of a chronic inflammatory condition. Thus, by monitoring the patient for expression patterns of SNX9, medical staff may become away of a regression or recurrence of the diseases earlier than they currently are, and consequently, provide earlier and more effective treatment.

Of course, more samples taken in more time-points may provide a statistically robust analysis of said expression trends, and may also be utilized as a method for continuous monitoring of subjects, especially those still undergoing and those that have undergone therapy. The more samples are available over a given time period, the higher is the resolution of the expression patterns of SNX9 during said period.

The number of samples collected and used for evaluation of the subject may change according to the frequency with which they are collected. For example, the samples may be collected at least every day, every two days, every four days, every week, every two weeks, every three weeks, every month, every two months, every three months every four months, every 5 months, every 6 months, every 7 months, every 8 months, every 9 months, every 10 months, every 11 months, every year or even more. Furthermore, to assess the trend in expression rates according to the invention, it is understood that the rate of change may be calculated as an average rate of change over at least three samples taken in different time points, or the rate may be calculated for every two samples collected at adjacent time points. It should be appreciated that the sample may be obtained from the monitored patient in the indicated time intervals for a period of several months or several years. More specifically, for a period of 1 year, for a period of 2 years, for a period of 3 years, for a period of 4 years, for a period of 5 years, for a period of 6 years, for a period of 7 years, for a period of 8 years, for a period of 9 years, for a period of 10 years, for a period of 11 years, for a period of 12 years, for a period of 13 years, for a period of 14 years, for a period of 15 years or more.

Still further, the invention provides a prognostic tool for detecting responders vs. non-responders to a given immune-based therapy.

The present invention relates to the diagnosis and monitoring of subjects or patients, in need thereof. By "patient" or "subject in need" it is meant any organism who may be affected by the above-mentioned conditions, and to whom the monitoring and diagnosis methods herein described is desired, including humans, domestic and non-domestic mammals such as canine and feline subjects, bovine, simian, equine and murine subjects, rodents, domestic birds, aquaculture, fish and exotic aquarium fish. It should be appreciated that the diagnosed or monitored subject may be also any reptile or zoo animal. More specifically, the methods of the invention are intended for mammals. By "mammalian subject" is meant any mammal for which the proposed therapy is desired, including human, livestock, equine, canine, and feline subjects, most specifically humans.

According to an aspect of some embodiments of the present invention there is provided a method of selecting a treatment regimen for treating a subject diagnosed with a chronic inflammatory condition, the method comprising: (a) evaluating the efficacy of a treatment with a therapeutic agent given to a subject suffering from a chronic-inflammatory condition according to the method of some embodiments of the invention, and (b) selecting a treatment regimen based on the evaluation; thereby selecting the treatment regimen for treating the subject diagnosed with said chronic inflammatory condition. In yet another embodiment, the invention provides a method of treating of a subject diagnosed with a chronic inflammatory condition, by evaluating the efficacy of a treatment, specifically, either a direct or indirect anti-inflammatory treatment, and selecting a treatment regimen based on the evaluation.

A third aspect of the invention relates to a diagnostic and prognostic kit for detecting and monitoring chronic inflammation and associated immune-suppression in a mammalian subject. The kit of the invention comprises:
(a) detecting molecules specific for determining the level of expression of SNX9 in a biological sample; and
(b) detecting molecules specific for determining the level of expression of at least one control reference protein in a biological sample.

Optionally, as an additional element (c), the kit of the invention may include at least one control sample. It should be noted that in certain embodiments, the control sample may be either a "negative" or a "positive" control. More specifically, a "negative" control may be a sample obtained from a subject that dose not display chronic inflammation. Such subject may occasionally referred to herein, as a "healthy" or "normal" subject. A "positive" control may be a sample obtained from a subject displaying chronic inflammation.

In certain optional embodiments, the kit of the invention may comprise as an additional element (d), instructions for carrying out the detection and quantification of expression of the biomarker of the invention, SNX9 and of at least one said control reference in the tested sample. Such instruction may also indicate procedure for obtaining an expression value of said SNX9 in said sample.

In other embodiments, the kit of the invention may optionally further comprise (e) predetermined calibration curve providing normalized standard expression values of said SNX9.

Still further, the kit of the invention may optionally further comprise (f), instructions for comparing the expression values of SNX9 in the test sample with a corresponding predetermined standard expression value according to (e) or with an expression value of SNX9 obtained from a control sample according to (c).

A combined use of SNX9 with other biomarkers is applied, the kit of the invention further comprises:
(a). detecting molecules specific for determining the level of expression of SNX18 in a biological sample;
(b). detecting molecules specific for determining the level of expression of TCR ζ chain in a biological sample; and
(c). detecting molecules for determining MDSCs population in a biological sample.

In certain embodiments, the detecting molecules provided with the kit of the invention may be isolated detecting amino acid molecules or isolated detecting nucleic acid molecules.

According to specific embodiments, the detecting molecules used by the kit of the invention are detecting amino acid molecules, specifically, an isolated antibody that specifically recognizes and binds SNX9.

In certain embodiments, where the level of expression of SNX9 is determined at the protein level, an immunoassay selected from the group consisting of FACS, a Western blot, an ELISA, a RIA, a slot blot, a dot blot, immunohistochemical assay and a radio-imaging assay is used. Therefore, the kit of the invention may further comprise reagents required for performing said assays.

The kit of the invention further comprises detecting amino acid molecules such as any one of isolated antibody that specifically recognizes and binds SNX18, an isolated antibody that specifically recognizes and binds TCR ζ chain, an isolated antibody that specifically recognizes and binds CD11b and an isolated antibody that specifically recognizes and binds Gr1.

According to certain alternative embodiments, the detecting molecules for SNX9 expression are isolated detecting nucleic acid molecules. According to some embodiments, such detecting nucleic acid molecules may be isolated oligonucleotides, each oligonucleotide specifically hybridizes to a nucleic acid sequence of the RNA products of said SNX9. More specifically, the oligonucleotide used as a detecting molecule according to certain embodiments of the invention may be any one of a pair of primers or nucleotide probe. In such case, the level of expression of SNX9 may be determined using a nucleic acid amplification assay selected from the group consisting of: a Real-Time PCR, micro arrays, PCR, in situ Hybridization and Comparative Genomic Hybridization. In certain embodiments, where the nucleic acid based detecting molecule is an aptamer, the detection of SNX9 expression is performed at the protein level.

As noted above, the kit of the invention comprises detecting molecules for at least one reference control protein. According to certain embodiments, such reference control may be any one of CD3ε, CD3δ, CD3γ, TCRα and TCRβ, for T cells, CD19 for B cells, SNX27 for all.

It should be noted that in certain embodiments, the kit of the invention may comprise detecting molecules specific for CD3ε that is used as the reference control protein. In yet another specific embodiment, CD19 is used as a reference control, specifically for B cells. Still further, anther embodiment of the invention relates to SNX27 as a control reference. According to certain embodiments, the kit of the invention is particularly suitable for detecting, monitoring and prognosting chronic inflammation in a subject suffering from a chronic inflammatory condition.

According to certain embodiments, the autoimmune disease may be an inflammatory arthritis.

According to another embodiment, the kit of the invention may be applicable in cases that the tested subject is suffering from a proliferative disorder, for example, any one of melanoma, carcinoma sarcoma, glioma, leukemia and lymphoma. More specific embodiments relate to melanoma and colorectal carcinoma.

It should be appreciated that the kit of the invention is suitable for determining the expression level of SNX9 in a biological sample. In some embodiments the biological sample may be any one of a whole blood sample, blood cells, bone marrow, lymph fluid, Spleen lymph nodes tissue samples, serum, plasma, urine, sputum, saliva, faeces, semen, spinal fluid or CSF, the external secretions of the skin, respiratory, intestinal, and genitourinary tracts, tears, milk, any human organ or tissue, any sample obtained by lavage optionally of the breast ductal system, plural effusion, samples of in vitro or ex vivo cell culture and cell culture constituents.

According to specific embodiments, the biological sample may be a blood sample. The kit of the invention may therefore optionally comprise suitable mans for obtaining said sample. More specifically, for using the kit of the invention, one must first obtain samples from the tested subjects. To do so, means for obtaining such samples may be required. Such means for obtaining a sample from the mammalian subject (a) can be any means for obtaining a sample from the subject known in the art. Examples for obtaining e.g. blood or bone marrow samples are known in the art and could be any kind of finger or skin prick or lancet based device, which basically pierces the skin and results in a drop of blood being released from the skin. In addition, aspirating or biopsy needles may be also used for obtaining spleen lymph nodes tissue samples. Samples may of course be taken from any other living tissue, or body secretions comprising viable cells, such as biopsies, saliva or even urine.

It should be appreciated that the kit of the invention may be applicable for monitoring and assessing responsiveness of a subject suffering from a chronic-inflammatory condition to a treatment with a therapeutic agent. In such case, the kit may further comprise as a further element (g), instructions for calculating the rate of change of the expression values (preferably, normalized values) of said SNX9 between said temporally-separated test samples. It should be noted that a positive rate of change of said expression values in a sample obtained after initiation of said treatment as compared to the SNX9 expression value in a sample obtained prior to initiation of said treatment, is indicative of the responsiveness of said subject to said treatment, that leads either directly or indirectly to an anti-inflammatory effect.

The inventors consider the kit of the invention in compartmental form. It should be therefore noted that the detecting molecules used for detecting the expression levels of SNX9 may be provided in a kit attached to an array. As defined herein, a "detecting molecule array" refers to a plurality of detection molecules that may be nucleic acids based or protein based detecting molecules (specifically, antibodies), optionally attached to a support where each of the detecting molecules is attached to a support in a unique pre- selected and defined region.

For example, an array may contain different detecting molecules, such as specific antibodies or primers. As indicated herein before, in case a combined detection of SNX9 expression level and in addition, of at least one of SNX18 or TCRζ chain, the different detecting molecules for each target may be spatially arranged in a predetermined and separated location in an array. For example, an array may be a plurality of vessels (test tubes), plates, micro-wells in a micro-plate, each containing different detecting molecules, specifically, antibodies, against SNX9 and at least one of SNX18 or TCRζ chain. An array may also be any solid support holding in distinct regions (dots, lines, columns) different and known, predetermined detecting molecules.

As used herein, "solid support" is defined as any surface to which molecules may be attached through either covalent or non-covalent bonds. Thus, useful solid supports include solid and semi-solid matrixes, such as aerogels and hydrogels, resins, beads, biochips (including thin film coated biochips), microfluidic chip, a silicon chip, multi-well plates (also referred to as microtiter plates or microplates), membranes, filters, conducting and nonconducting metals, glass (including microscope slides) and magnetic supports. More specific examples of useful solid supports include silica gels, polymeric membranes, particles, derivatized plastic films, glass beads, cotton, plastic beads, alumina gels, polysaccharides such as Sepharose, nylon, latex bead, magnetic bead, paramagnetic bead, superparamagnetic bead, starch and the like. This also includes, but is not limited to, microsphere particles such as Lumavidin.TM. or LS-beads, magnetic beads, charged paper, Langmuir-Bodgett films, functionalized glass, germanium, silicon, PTFE, polystyrene, gallium arsenide, gold, and silver. Any other material known in the art that is capable of having functional groups such as amino, carboxyl, thiol or hydroxyl incorporated on its surface, is also contemplated. This includes surfaces with any topology, including, but not limited to, spherical surfaces and grooved surfaces.

It should be further appreciated that any of the reagents, substances or ingredients included in any of the methods and kits of the invention may be provided as reagents embedded, linked, connected, attached, placed or fused to any of the solid support materials described above.

Therefore, in a further aspect, the present invention provides diagnostic and prognostic kits and methods for the detection and monitoring of chronic inflammation, in a mammalian subject suffering from a chronic inflammatory condition. Such conditions include autoimmune disorders such as arthritis, proliferative disorders such as melanoma and colorectal carcinoma, and infectious diseases. More specifically, the diagnostic methods and kits provided by the invention use as a marker for chronic inflammatory disorders or conditions, the level of SNX9 in the tested biologic sample. Reduced levels of SNX9 in the tested sample as compared to the levels in a healthy control, indicates that the sample is of a subject suffering from chronic inflammation. It should be appreciated that the diagnostic kits and methods of the invention further provide a tool for a "tailor-made" or personalized therapy, by identifying subjects suffering from a specific chronic inflammation that are likely to be benefit from treatment with an anti-inflammatory therapeutic agent.

Taken together, diagnostic and prognostic agents of the present invention (e.g., oligonucleotide and antibodies, described above) can be packaged in a diagnostic kit. Such diagnostic kits can include an antibody (e.g., labeled) of the present invention in one container and a solid phase for attaching multiple biological samples packaged in a second container as well as imaging reagent in a third container (e.g., secondary labeled antibody) with appropriate buffers and preservatives and used for diagnosis.

In certain embodiments, the compositions of the invention may further comprise detecting molecules specific for control reference protein. Such reference protein may be used for normalizing the detected expression levels for the biomarker of the invention SNX9. According to one optional embodiment, the compositions described by the invention or any components thereof, specifically, the detecting molecules may be attached to a solid support. The solid support may include polymers, such as polystyrene, agarose, sepharose, cellulose, glass, glass beads and magnetizable particles of cellulose or other polymers. The solid-support can be in the form of large or small beads, chips or particles, tubes, plates, or other forms.

As shown by Example 1, the inventors were first to show that SNX9 is associated with TCR (T cell receptor) and moreover, regulates TCR expression, cap formation, surface expression and internalization. These findings clearly define the role of SNX9 as a key immunomodulator. Moreover, these results indicate that SNX9 may serve as a target molecule for immuno-modulation, specifically, in treating immune-related disorders.

All scientific and technical terms used herein have meanings commonly used in the art unless otherwise specified. The definitions provided herein are to facilitate understanding of certain terms used frequently herein and are not meant to limit the scope of the present disclosure.

As used herein the term "about" refers to ± 10 % The terms "comprises", "comprising", "includes", "including", "having" and their conjugates mean "including but not limited to". The term "consisting essentially of" means that the composition, method or structure may include additional ingredients, steps and/or parts, but only if the additional ingredients, steps and/or parts do not materially alter the basic and novel characteristics of the claimed composition, method or structure.

The term "about" as used herein indicates values that may deviate up to 1%, more specifically 5%, more specifically 10%, more specifically 15%, and in some cases up to 20% higher or lower than the value referred to, the deviation range including integer values, and, if applicable, non-integer values as well, constituting a continuous range.

As used herein the term "about" refers to ± 10 %. The terms "comprises", "comprising", "includes", "including", "having" and their conjugates mean "including but not limited to". This term encompasses the terms "consisting of' and "consisting essentially of'. The phrase "consisting essentially of' means that the composition or method may include additional ingredients and/or steps, but only if the additional ingredients and/or steps do not materially alter the basic and novel characteristics of the claimed composition or method. Throughout this specification and the Examples and claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

It should be noted that various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible sub ranges as well as individual numerical values within that range. For example, description of a range such as from 1 to 6 should be considered to have specifically disclosed sub ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

Various embodiments and aspects of the present invention as delineated hereinabove and as claimed in the claims section below find experimental support in the following examples.

Disclosed and described, it is to be understood that this invention is not limited to the particular examples, methods steps, and compositions disclosed herein as such methods steps and compositions may vary somewhat. It is also to be understood that the terminology used herein is used for the purpose of describing particular embodiments only and not intended to be limiting since the scope of the present invention will be limited only by the appended claims and equivalents thereof.

### EXAMPLES

Reference is now made to the following examples, which together with the above descriptions, illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley; Sons, New York (1988); Watson et al., "Recombinant DNA", Scientific American Books, New York; Birren et al. (eds) "Genome Analysis: A Laboratory Manual Series", VoIs. 1-4, Cold Spring Harbor Laboratory Press, New York (1998). "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. L, ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader.

### Mice

Female/male BALB/c, DBA/1, B10.A and C57BL/6 mice, 6 to 8 weeks of age, were bred at the Hebrew University specific pathogen-free facility. Animal use followed protocols approved by the Hebrew University-Hadassah Medical School Institutional Animal Care and Use Committee.

### Animal models

### Chronic inflammation:

Normal C57BL/6 mice repeatedly exposed to heat-killed BCG were used as the mouse model for chronic inflammation¹. BCG was administered by three subcutaneous injections at 1wk intervals, 100 µg per animal/dose; the first two doses were administrated with IFA (Incomplete Freund's adjuvant), and subsequent injections included heat-killed BCG in PBS. Unless stated otherwise, splenocytes were collected 2-3 days after the last injection. Control mice remained untreated. For experiments with chicken OVA (Sigma) as antigen, the same protocol was maintained: For a Th1 response BCG was administered as above but with OVA. For a Th2 response, the first and second injections were with 100 µg OVA adsorbed to Al(OH)3 (alum; Thermo), and the third injection was with 100 µg OVA in PBS. Experimental groups included at least three mice, and each experiment was repeated at least three times.

### Lehishmania infected mice:

The left hind footpads of 8 weeks BALB/c mice were inoculated with 10 x 10⁶ stationary phase culture of L. donovani promastigotes in 40 µl PBS intraperitonealy ²⁵. Mice were sacrificed three months post infection and splenocytes were collected for quantifying parasite burden and for FACS and Western analysis.

### Tumor bearing mice:

A mouse melanoma cell line B16 (clone F10.9) ^{26, 27} was maintained in RPMI-1640 medium supplemented with 10% fetal bovine serum, 2 mM L-glutamine, 0.75% sodium bicarbonate, 10 µM HEPES buffer, 50 Units/mL penicillin-streptomycin and 25 µg /mL gentamicin. Cells (1 x 10⁶ in a final volume of 0.1 ml) were injected s.c. into the right rear flank of 6-8 week old male and/or female C57BL/6 mice. Animals were weighed twice weekly and observed daily. Approximately 20 days after inoculation of the B16-F10 cells, or when the tumor size was 10 mm, the mice were sacrificed and the tumor masses were dissected from the implantation site and the actual tumor size measured (average weight of each tumor was 0.1gr) and splenocytes were collected.

### Colon carcinoma bearing mice:

Female C57BL/6 mice were injected intraperitoneally with 12 mg/kg body weight of azoxymethane (AOM) dissolved in physiological saline. Five days later, 2% dextran sulfate sodium (DSS) was given in the drinking water over 5 days, followed by 16 days of regular water²⁸. This cycle was repeated a total of three times. Body weight was measured every week, and the animals were sacrificed two weeks after the third cycle for macroscopical inspection and histological analysis.

### Rheumatoid arthritis- collagen induced arthritis mouse model:

Male DBA/1 mice (8-12 weeks old) were injected intradermaly at the base of the tail with 200 µg type II collagen purified from bovine articular cartilage and emulsified in complete Freund's adjuvant. The mice received a booster injection of 200 µg type II collagen emulsified in CFA, 3 weeks after the first dose ²⁹. The mice were inspected daily with microcalipers and each animal with erythema and/or swelling in one or more limbs was randomly assigned to one of several groups, which sacrificed on the day of disease onset (day 0), disease peak (day 5), first disease extinguishing (disease peak + 16 days) or at the second disease extinguishing (disease peak + 30 days). On the day of sacrifice, splenocytes were collected for FACS and western blot analysis. Unless otherwise indicated. Arthritis was monitored over a 10- to 21-day treatment period in terms of the criteria of the disease.

### Experimental procedures

### In vivo 5-fluorouracil (5FU) chemotherapeutic treatments

BCG-treated mice received three i.p. injections of 5-fluorouracil (5FU); The first 5FU injection (1mg/mouse) was given at the day of the second BCG treatment and the second 5FU injection was given four days later. The third 5FU injection (0.5 mg/mouse) was given at the day of the third BCG treatment.

### Cell isolation and separation

For magnetic column separation, splenocytes were first labeled (30 minutes at 4°C) with biotin-conjugated antibodies. Negative selection using anti-CD11b, anti-Grl and anti-B220 antibodies was performed for T-cell isolation (> 92% purity), and positive selection was performed for the separation of Gr-1+Mac-1+ cells using anti-Gr-1 antibodies (> 98% purity). The cells were then washed, labeled (45 minutes at 4°C) with anti-biotin antibodies conjugated to magnetic microbeads (Miltenyi Biotec, Auburn, CA), washed, and loaded onto a column placed in a magnetic field (Miltenyi Biotec). For separation of B cells, the same positive selection protocol was applied using CD43 magnetic microbeads (Miltenyi Biotec, Auburn, CA).

### T Cell Stimulation

For T cell activation, 5x10⁷/ml EL4 or 2X10⁶/ml C57BL6 or isolated T splenic cells maintained in RPMI medium 1640 supplemented with 10% FCS and 2 mM 1-glutamine penicillin/streptomycin, were stimulated for varying times with anti-CD3 (1 µg/ml) and/or anti-CD28 (1 µg/ml) antibodies. Cell activation was confirmed by anti-phosphotyrosine immunoblotting of lysate aliquots or staining for CD25 using FACS analysis.

### Ex vivo cell co-culture system

EL4 T cell line, T or B cells isolated from normal spleen were suspended in complete growing medium and co-incubated for 16 hours at 37°C with a Gr1+Mac-1+-enriched cell population obtained from the spleen of BCG-treated mice. The cells were then harvested and subjected to lysis, resolved on SDS/PAGE and subjected to immunoblotting with anti-SNX9, anti-SNX27, anti-ζ-chain and CD3ε antibodies.

### Immunostaining and FACS analysis

The antibodies used for cell surface labeling were FITC-labeled anti-Thy-1.2 and PE-labeled anti-CD45R/B220, and biotinylated anti-CD3ε and anti-TCRαβ, these were detected by streptavidin-Cy5 (Jackson Immunoresearch). For humane markers APC-labeled anti-CD3 and FITC-labeled anti-CD19 Ab, were used. All the antibodies were purchased from Biolegend. Cells were pre-coated with anti-mouse CD16/CD32 and then incubated for 30 min at 4 °C with the specific labeled antibodies. After being washed, the relevant samples were incubated with a second-step reagent. For intracellular staining of the ζ and CD3ε chains, the cells were first stained for the cell surface T cell marker, then were washed, fixed for 20 min with 1% paraformaldehyde at 4°C and permeabilized for 10 min at room temperature with 0.1% saponin diluted in FACS buffer. Cells were washed and then incubated for 30 min with biotin-labeled anti-CD3ε (BD Pharmingen) or with biotinylated monoclonal anti-ζ (H146); these were detected with streptavidin-Cy5 (Jackson Immunoresearch). Samples were analyzed in a FACSCalibur apparatus with Cell Quest software (BD) or under ImageStreamx system (Amnis) for TCR/CD3 capping analysis.

### Lysis, immunoprecipitation and immunoblotting

Analyses used either the splenocyte population or EL4 T cell line. Cells (5 x10⁷/ml) were lysed with Tris-NaCl buffer containing 0.5% Triton X-100 and protease inhibitors for 30 min on ice. Proteins were resolved on 12% SDS-PAGE and subjected to immunoblotting analysis using specific antibodies: anti-ζ, anti-SNX9, anti-SNX27 and anti-CD3ε antibodies. Specific antibodies were detected by incubation with protein A (Amersham), anti-rat or anti-goat antibodies conjugated to horseradish peroxidase (Jackson Immunoresearch), followed by enhanced chemiluminescence and exposure to Kodak X-ray films. For immunoprecipitation, cell lysates were incubated for 3 hours with Protein A/G sepharose beads (Amersham) pre-coated with specific antibodies at 4°C, beads were washed, samples were eluted and subjected to immunoblotting using the appropriate antibodies.

### Real Time PCR, a Quantitative PCR

Total RNA was recovered from splenocytes or isolated MDSCs using Tri-Reagent (Sigma) and was subjected to reverse transcription with m-MLV-RT (Invitrogen) and random primers (IDT). Quantitative mRNA expression was analyzed by real-time PCR (ABI 7900), with SYBR green (Invitrogen). RT-PCR primers were designed to recognize an exon-exon boundary in all transcripts.

### The following primers were used:

**A forward primer:** AACAGTGTGATCCGCCTCTACC (mouse origin), as also denoted by SEQ ID NO. 1.

**A reverse primer:** TGAAGCACTTCGGAGTTCTCTTC (mouse origin), as also denoted by SEQ ID NO. 2.

### DNA Constructs and Transfection of COS-7 Cells

COS-7 cells were grown in Dulbecco's modified Eagle's medium supplemented with 10% fetal calf serum (FCS) and transfected using DEAE-Dextran with the mouse full length, proximal and distal ζ cDNA or with CD3ε cDNA that were cloned into pCDNA3.1 vector (the sequence was verified³).

Cells were harvest and the pellets were lysed by using Tris lysis buffer containing 0.5% Triton X-100 and subjected to immuno-blotting.

### TCR/CD3ε internalization assay

EL4 T cells were incubated for 30 min on ice with anti-CD3ε antibody (1 µg/ml) followed by biotinylated goat anti-hamster antibody (2 µg/ml) for another 30 min on ice. Cells were then washed and treated with 0.1% NaN3 on ice or warmed to 37°C for 2, 5, 10, or 15 min to allow internalization. Cells were then treated with 0.1% NaN3 on ice and stained with Cy5-conjugated streptavidin, washed, and subjected to FACSCalibur analysis. The data were expressed as the median of the population and graphed as the percentage change in surface level CD3ε over the time course.

### SNX9 protein knockdown in EL4 cells

SNX9 knockdown was achieved using shRNA GFP lentiviral vectors (kindly gift from our collaborator Lichtenthaler SF, DZNE-German Center for Neurodegenerative Diseases Munich, 80336 Munich, Germany). A non-targeting shRNA (composed of non-targeting shRNA) was used to assess non-specific effects of shRNA delivery. High-titer lentiviral vectors were generated by transfecting 10-14-cm plates of HEK 293T cells with the lentiviral backbone along with the packaging plasmids (gag-pol and pMD.G) using the BBS/calcium phosphate transfection reagent (50 mM BES, 280 mM NaCl, 1.5 mM Na₂HPO₄ at pH 6.95, 0.25 M CaCl₂). Supernatant from 48 hours transfected HEK 293T cell was harvest and incubated with EL4 cell for additional 16 hours. Knockdown of SNX9 protein in EL4 cell was visualized by GFP positive cells using FACS analysis and verified by Western blotting.

### Example 1

### The involvement of SNX9 in TCR regulation under normal conditions

### SNX9 and the ζ chain are associated

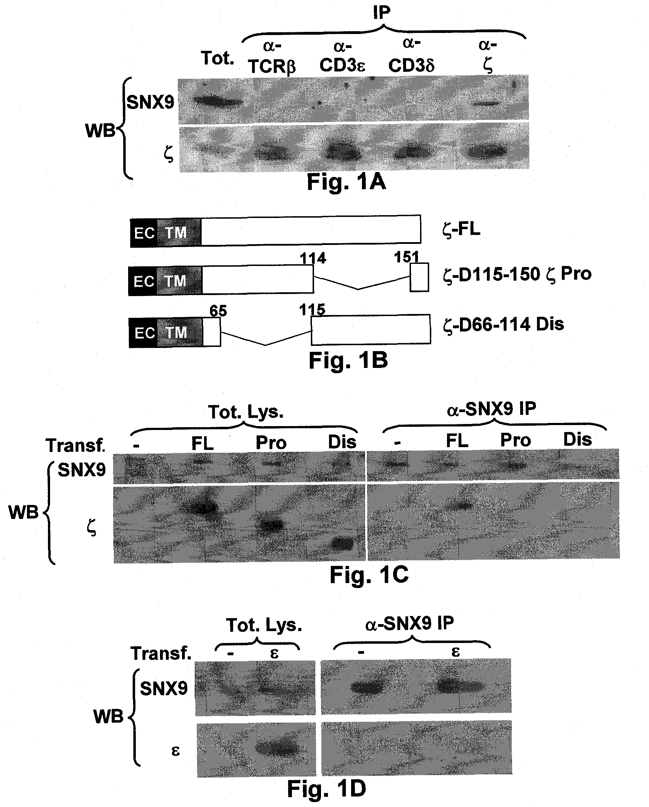

The inventors aimed at investigating the involvement of SSX9 in TCR regulation under normal conditions. Therefore, the interaction between SNX9 and TCR ζ chain was first examined. As clearly demonstrated by Figure 1A, using specific antibodies in co-immunoprecipitation analyses, an association between SNX9 and the TCR via the ζ chain, was observed. Only anti-ζ antibodies precipitate SNX9 but not antibodies directed against the reminder TCR subunits (Fig. 1A). As shown by Figures 1B and 1C, SNX9 associates only with the full-length ζ chain and not with truncated ζ chains or the CD3ε chain (Fig. 1D). This was shown when using immunoprecipitations of lysates from COS cells that highly express high endogenous levels of SNX9, transfected with full-length or truncated ζ chains or with the CD3ε.

### SNX9 regulates cell surface TCR expression

In order to assess the effect of SNX9 on TCR expression, the inventors generated SNX9 KO (knock out) T cell line by using shRNA for SNX9, and analyzed the TCR features in these cells. As shown by Figure 2A, expression of SNX9 was not detected in these KO cells. Figure 2B demonstrates an increased cell surface TCR expression detected in the SNX9 KO cells. The inventors further observed a slower TCR internalization upon activation (Fig. 2C) compared with that observed SNX9 expressing cells. These results suggest a role for SNX9 in TCR cell surface expression and internalization.

### SNX9 affects TCR cap formation

The inventors next examined the effect of SNX9 on TCR capping upon CD3 stimulation. The preliminary data presented by Figure 3, indicate that SNX9 affects TCR capping upon TCR cross linking, which is a key process towards immunological synapse (IS) formation. As clearly shown by the figure, SNX9 OK cells display lower levels of TCR capping compared with cells expressing the WT SNX9.

### Increased SNX9 expression levels after activation of isolated T cells

The effect of TCR-mediated T cell activation on SNX9 expression was next analyzed in order to learn more about its regulation in the immune system. As shown by Figure 4A, activation of T cells using CD3/CD28 antibodies, increased the expression levels of SNX9 (upper panel), while ζ expression levels were slightly decreased (lower panel). The fact that in non-activated cells SNX9 expression levels decreased as demonstrated by the upper panel of Fig. 4B, indicates that activation-dependent SNX9 up-regulation was even more pronounced. These results together with those described in Figure 3 suggest that SNX9 controls TCR expression and function and vice versa; TCR-mediated signaling controls SNX9 expression.

### SNX9 expression in T cells and B cells

In the course of these studies the inventors observed that SNX9 is expressed in both T and B cells and in the latter, its expression is more abundant (Fig. 5). Interestingly, while SNX9 is considered ubiquitously expressed, the inventors found that it is absent in immature myeloid cells (MDSCs), which originate in the bone marrow and are also present in the spleen of chronically inflamed mice, as demonstrated by Figure 5.

Taken together, the observed SNX9 expression in isolated T and B cells, and the decreased SNX9 expression shown in non-activated isolated T cells hint at the possible role of SNX9 in homeostasis.

### Example 2

### SNX9 is uniquely regulated under pathological conditions characterized by chronic inflammation

Demonstrating the regulatory role of SNX9 in TCR expression and regulation in normal conditions, encouraged the inventors to examine the regulatory role of this molecule under pathologic conditions.

### An in vivo regulation of SNX9 during chronic inflammation

In the course of analyzing cells from a chronic inflammatory environment the inventors had previously demonstrated that TCRζ chain expression is down-regulated in T and NK cells. Examination of TCR ζ chain expression levels as presented by the lower panels of Figures 6A and 6B, clearly indicates that ζ chain expression levels are down-regulated as well, in the spleen and peripheral blood, while the expression of the rest of the TCR subunits remains normal (Fig. 6 A, B, third panel). Since SNX9 shares similar structural and functional domains with SNX18, the inventors tested the expression levels of the latter under chronic inflammatory conditions and found that it is down regulated as well. Interestingly, as demonstrated by the upper panels of Figures 6A and 6B, SNX9 and SNX18 are more dramatically down regulated when compared to the ζ chain. Moreover, the observed SNX9 and ζ chain down-regulation are observed only in the course of a chronic Th1-mediated inflammatory response and not during a Th2-mediated chronic response (Fig. 6C). Figure 6D demonstrates the option of using SNX27 as a control reference since this protein is not sensitive to inflammatory status (see increasing SNX27 levels, upper panel).

As shown by the figure, SNX9 down regulation is apparent at a ratio of 4(T): 0.5(MDSCs) while ζ is not down regulated yet. Usually, ζ chain is down regulated at a ratios of 4(T):8(MDSCs). Indicating that SNX9 is more sensitive to the immunosuppressive environment. Additional experiments indicate that ζ is more sensitive to the recovery state, upon treatment.

It should be noted that the reduced levels of SNX9 in chronic inflammation was observed also when the expression was examined by Real-time PCR. More specifically, as demonstrated by Figure 6E, the levels of SNX9 mRNA from total splenic normal mice, BCG treated mice and isolated MDSC's were quantified by quantitative RT-PCR as described in Methods, and normalized to the level of *Tubulin* mRNA. **P* < 0.05. It is important to note that some SNX9 mRNA is detected in the MDSCs while at the protein levels it is not seen. In any event SNX9 expression levels are significantly decreases in the BCG-treated mice

### Example 3

### Down-regulation of SNX9 expression under chronic inflammatory environment correlates with the elevated levels of MDSCs and is a reversible phenomenon

Kinetic experiments presented by the upper panel of Figure 7A revealed that SNX9 is down regulated at the chronic inflammatory state, similar to the ζ chain. However, its down-regulation is much more pronounced than that of the ζ chain. Moreover, upon recuperation of the inflammatory environment SNX9 expression levels start to recover a similar feature as the ζ chain (Fig. 7A, upper panel) although at a slower rate, indicating a reversible phenomenon. It is important to note that under conditions of chronic inflammation-induced immunosuppression the down-regulation of SNX9 expression is completely depleted from the entire splenic population. While the down-regulation was observed in the spleen (Fig 7A, upper panel) and peripheral blood (Fig. 6), in the lymph nodes SNX9 expression was steady, similar to what the inventors had previously shown for the ζ chain (Fig 7B, upper panel). SNX9 down-regulation directly correlates with the MDSCs levels as shown in Figs. 7A and 7B, lower panels. Due to the absence of MDSCs in the lymph nodes, SNX9 expression remains unchanged. Thus, SNX9 behavior is similar to the ζ chain but with a different sensitivity to the chronic inflammatory environment.

### Example 4

### SNX9 down-regulation under chronic inflammatory conditions is mediated via MDSCs:

Using *ex vivo* co-incubation experiments, the inventors now demonstrate that the massive down modulation of SNX9 in B and T cells is caused by Gr1⁺Mac1⁺ MDSCs (Fig. 8A,B), while the latter do not express SNX9 compared with B and T cells (Fig. 8A,B). These results point at the potential use of SNX9 expression levels as a biomarker for evaluation of the immune status, detecting chronic inflammation and associated immuno-suppression. Moreover, based on the reversible features of SNX9 down-regulation it could also serve as a biomarker for measuring efficacy of given therapy that leads directly or indirectly to the recovery of the chronic inflammatory environment.

The inventors next tested whether changes in SNX9 expression levels are also evident in various pathologies characterized by chronic inflammation and reflect the hosts' immune status. To this end, the inventors tested mouse models for diseases characterized by chronic inflammation as autoimmune diseases (rheumatoid arthritis), cancer (melanoma and colorectal carcinoma) and infections (Leishmania).

### Example 5

### SNX9 is down regulated in the course of rheumatoid arthritis (RA)

Collagen-induced arthritis, generated in DBA/1 mice following inoculation with type II collagen and CFA ²⁹, resembles human RA in clinical manifestations, histopathological profile, immunological mechanism and genetic susceptibility. RA is an autoimmune disease characterized by chronic inflammation of multiple joints, ultimately leading to cartilage and bone erosion and loss of joint function ²⁹. The inventors next tested whether SNX9 expression levels are affected at different stages of the disease and compared its expression to that of the ζ chain. Four groups were assessed: (1). Mice injected with collagen and CFA and tested during the onset and pick disease stages; (2). Mice tested upon disease recovery: 16 and 30 days after the observed disease pick; (3). Mice subjected only to CFA injections; and (4). Normal mice. Mice were sacrificed and splenic cells were analyzed by immunoblotting for SNX9, CD3ε and ζ expression levels (Fig. 9A). As demonstrated by Figure 9A, the results point at the dramatic SNX9 down-regulation already at the onset as well as in the pick stage of the disease and the slow recovery to normal, only 30 days after the disease pick. In contrast, the ζ chain down-regulation and recovery were milder while CD3ε expression levels were unchanged. When looking at the experimental group subjected to CFA only, clear SNX9 down-regulation at the onset stage and full recovery at the pick stage was observed. These results indicate few major points: (a) that the inflammatory response generated in CIA leads to SNX9 down-regulation much earlier and more extensive when compared to the ζ chain and it recovers upon dilution of the inflammatory environment. (b) SNX9 down-regulation is reversible, (c) CFA can induce SNX9 down-regulation but it is transient; when Collagen is injected with CFA a severe disease develops and the down-regulation is holding in the pick stage and thereafter. In parallel to the Western blot analysis, part of the cell suspension harvested from the different experimental groups was subjected to FACS analysis for the detection of Gr1⁺ Mac1⁺ double positive cells (Fig. 9B). As can be seen, SNX9 down-regulation and recovery inversely correlates with the elevated levels of MDSCs similar to what was observed in the model system for chronic inflammation described above.

These results point at the possible use of SNX9 expression levels as a biomarker for measuring the immune status in RA, evaluating the development of chronic inflammation and associated immuno-suppression. Due to the reversible features of SNX9 down-regulation depending on the severity of the inflammatory environment, evaluation of SNX9 expression levels could also serve as a biomarker for measuring efficacy of given therapy that leads directly or indirectly to the recovery of RA and accordingly of the chronic inflammatory environment.

### Example 6

### B16 melanoma induces SNX9 down-regulation

As mentioned above, the inventors have shown that chronic inflammation is the cause for immunosuppression as observed in human and mice with developing tumors; MDSCs are accumulated, leading to T and NK cell dysfunction associated with ζ degradation. Moreover, the inventors also demonstrated that the immunosuppressive environment negatively affects newly administered T cells, and thus could limit the success of cancer immunotherapy based on vaccination, T cell transfer and antibody-mediated therapy. There is an urgent need for biomarkers to evaluate the host's immune status prior to a given immunotherapy and to indicate whether the inflammatory environment has to be neutralized prior to the given therapy. Moreover, such biomarkers could be also used in the evaluation of a given therapy; whether the tumor regresses or reappears based on the developing chronic inflammatory environment. During the past decade the inventors discovered that ζ chain expressed in T, NKT and NK cells could serve as such a biomarker. Now, the inventors identified that SNX9, which is also expressed in B cells, as an additional biomarker that plays a role in a pathway different from that of the ζ chain, for evaluating the immune status under chronic inflammatory conditions. The inventors thus aimed to define whether SNX9 expression levels are also affected during tumor development. To this end, the mouse model system for melanoma (B16-F10.9) tumors growing subcutaneously ²⁷ was used. The tested tumors were subcutaneously growing with no detected metastases. In these mice splenocytes were first analyzed for SNX9, ζ chain and CD3ε expression levels by immunoblotting as shown by Figure 10A. The results revealed that, while at this stage of tumor growth, ζ chain expression levels were slightly down regulated, SNX9 expression was significantly decreased. SNX9 down-regulation was inversely correlated with the levels of MDSCs (Fig. 10B, 10C) similar to the ζ chain. It is important to note that SNX9 expression levels are more sensitive to the minute changes of the chronic inflammatory environment. However, ζ chain expression levels are more sensitive to the recovery of the environment, potential use of both biomarkers simultaneously.

To examine the feasibility of using SNX9 as a biomarker for proliferative disorders such as melanoma, the inventors next examined the expression of this protein in blood samples obtained from melanoma patients. Whole blood samples obtained from three melanoma patients and three healthy donors were lysed and subjected to immunoblotting analysis as mentioned in the "Experimental Procedures". The expression levels of CD3ε, ζ chain and SNX9 were analyzed using specific antibodies directed against each protein. SNX9 and ζ expression levels were normalized to CD3ε expression, which is unchanged under the same conditions. At the second step SNX9 and ζ expression levels were normalized to the expression of each in healthy donors. The normal levels of CD3ε expression and reduced level of ζ chain expression were validated by FACS analysis. Figure 11C discloses a graph summarizing Western-blot analysis profiles performed on triplicate donors in each experimental group for the protein expression levels of CD3ε, ζ chain and SNX9 in the peripheral blood of healthy donors and melanoma patients. As clearly indicated by the figure, blood samples of melanoma patients show reduced levels of SNX9 expression.

Whole blood samples obtained from the same healthy and melanoma donors were stained for total CD3 and CD19 expression. Levers of expression were measured by FACS as the mean fluorescent intensity (MFI). As sown by Figures 11A and 11B, no statistical significant difference in CD3 expression was observed within T cells derived from melanoma patients relative to healthy donors and in B cells stained with a CD 19 antibody. These results demonstrate the specificity of the SNX9 down regulation observed in Figure 11C.

### Example 7

### Colorectal cancer induces SNX9 down-regulation

The inventors next examined the possible use of SNX9 as a biomarker for additional proliferative disorders, therefore, an animal model for colorectal carcinoma ²⁸ was next analyzed. Female C57BL/6 mice were injected intraperitoneally with 12 mg/kg body weight of azoxymethane (AOM) dissolved in physiological saline. Five days later, 2% dextran sulfate sodium (DSS) was given in the drinking water over 5 days, followed by 16 days of regular water. This cycle was repeated a total of three times. Body weight was measured every week, and the animals were sacrificed two weeks after the third cycle for macroscopical inspection and histological analysis.

Splenic cells were harvested from normal and from tumor-bearing mice after AOM and DSS treatment, as indicated. The cells were lysed, resolved on SDS/PAGE and subjected to immunoblotting using anti-SNX9, anti-CD3ε and anti-ζ antibodies. A representative experiment is presented by Figure 12. While the ζ chain under these conditions is slightly down regulated, if normalized to the CD3ε expression, the SNX9 expression levels are dramatically decreases (Figure 12A). Graphs presented by Figure 12B summarize FACS analysis profiles performed on triplicate mice in each experimental group for Gr1+Mac1+ double-positive MDSC cells from normal mice and from mice bearing colorectal tumors. As shown by the figure, samples from colorectal carcinoma bearing animals show increased MDSC cells population.

### Example 8

### Leishmania donovani infection induces SNX9 down-regulation

During active disease of *Leishmania donovani* in humans and mouse models, high levels of IFNγ and TNFα detected in the serum, suggest that an inflammatory immune response is highly activated ²⁵. Infection with *Leishmania donovani* results in the development of organ-specific immunity in the two main target tissues of infection, the spleen and the liver. The liver is the site of an acute resolving infection associated with the development of inflammatory granulomas around infected Kupffer cells, and resistance to re-infection. Paradoxically, the spleen is an initial site for the generation of cell-mediated immune responses, but ultimately becomes a site of parasite persistence with associated immunopathological changes ²⁵. These include splenomegaly and a breakdown in tissue architecture that is postulated to contribute to the immuno-compromised status of the host. In the course of the studies of the present invention looking at various pathologies with a different etiology and physiology but sharing a common denominator of chronic inflammation, the inventors aimed to define whether SNX9 expression levels are changed during *Leishmania donovani* infection. Moreover, the inventors tested the correlation between SNX9 expression levels, percentage of MDSCs and ζ expression. The results reveal that under the pathological conditions generated during three month of *Leishmania donovani* infection, SNX9 expression levels are down regulated and basically disappears (Fig. 13A) as compared to the ζ chain that is significantly down-regulated but to a lesser degree (Fig. 13A). The more pronounced effect on the ζ chain expression is at a later stage of infection (data not shown). The down-regulation of both biomarkers inversely correlates with MDSCs levels (Fig. 13B, 13C). Under this condition the CD3ε chain remains unchanged (Fig. 13A). Again, the dramatic SNX9 down-regulation reflects affected T and B cells.

### Example 9

### SNX9 expression levels 'sense' 5FU treatment, which eliminates MDSCs

Based on the cumulative data highlighting the SNX9 characteristics as being a potential biomarker for the evaluation of the hosts' immune status, the inventors aimed at testing its capacity to sense changes in the inflammatory environment upon chemotherapeutic treatment that affects MDSCs. 5FU was chosen since it was recently demonstrated as having a cytotoxic effect on MDSCs ³⁰

Mice displaying chronic inflammation were treated with various doses of 5FU at different intervals in the course of the inflammatory response. Herein the invention present on Figure 14, one of the tested conditions showing down regulation of SNX9 and ζ chain during chronic inflammation and associated immunosuppression and upon 5FU treatment at the chronic inflammatory state. As shown in Figure 14A, 5FU treatment led to the recovery of ζ and SNX9 expression levels inversely correlated with the MDSCs levels (Fig. 14B, C). These results clearly indicate that SNX9 as well as ζ or any combination thereof, can serve as biomarkers for the detection of the immune status and therefore demonstrates the feasibility of using SNX9 for monitoring efficacy of given therapies.

### References:

1. Bronstein-Sitton, N. et al. Sustained exposure to bacterial antigen induces interferon-gamma-dependent T cell receptor zeta down-regulation and impaired T cell function. Nat Immunol 4, 957-964 (2003).
2. Ezernitchi, A.V. et al. TCR zeta down-regulation under chronic inflammation is mediated by myeloid suppressor cells differentially distributed between various lymphatic organs. J Immunol 177, 4763-4772 (2006).
3. Vaknin, I. et al. A common pathway mediated through Toll-like receptors leads to T- and natural killer-cell immunosuppression. Blood 111, 1437-1447 (2008).
4. Kieper, W.C., Burghardt, J.T. & Surh, C.D. A role for TCR affinity in regulating naive T cell homeostasis. J Immunol 172, 40-44 (2004).
5. Liu, H., Rhodes, M., Wiest, D.L. & Vignali, D.A. On the dynamics of TCR:CD3 complex cell surface expression and downmodulation. Immunity 13, 665-675 (2000).
6. Call, M.E. & Wucherpfennig, K.W. The T cell receptor: critical role of the membrane environment in receptor assembly and function. Annu Rev Immunol 23, 101-125 (2005).
7. Dietrich, J. & Geisler, C. T cell receptor zeta allows stable expression of receptors containing the CD3 gamma leucine-based receptor-sorting motif. J Biol Chem 273, 26281-26284 (1998).
8. Szymczak, A.L. & Vignali, D.A. Plasticity and rigidity in adaptor protein-2-mediated internalization of the TCR:CD3 complex. J Immunol 174, 4153-4160 (2005).
9. Baniyash, M. TCR zeta-chain downregulation: curtailing an excessive inflammatory immune response. Nat Rev Immunol 4, 675-687 (2004).
10. Geisler, C. TCR trafficking in resting and stimulated T cells. Crit Rev Immunol 24, 67-86 (2004).
11. Janvier, K. & Bonifacino, J.S. Role of the endocytic machinery in the sorting of lysosome-associated membrane proteins. Mol Biol Cell 16, 4231-4242 (2005).
12. Bonifacino, J.S. & Rojas, R. Retrograde transport from endosomes to the trans-Golgi network. Nat Rev Mol Cell Biol 7, 568-579 (2006).
13. Alcover, A. & Alarcon, B. Internalization and intracellular fate of TCR-CD3 complexes. Crit Rev Immunol 20, 325-346 (2000).
14. Monjas, A., Alcover, A. & Alarcon, B. Engaged and bystander T cell receptors are down-modulated by different endocytotic pathways. J Biol Chem 279, 55376-55384 (2004).
15. Valitutti, S., Muller, S., Salio, M. & Lanzavecchia, A. Degradation of T cell receptor (TCR)-CD3-zeta complexes after antigenic stimulation. J Exp Med 185, 1859-1864 (1997).
16. Weissman, A.M. et al. Role of the zeta chain in the expression of the T cell antigen receptor: genetic reconstitution studies. EMBO J 8, 3651-3656 (1989).
17. Klausner, R.D., Lippincott-Schwartz, J. & Bonifacino, J.S. The T cell antigen receptor: insights into organelle biology. Annu Rev Cell Biol 6, 403-431 (1990).
18. Lundmark, R. & Carlsson, S.R. Regulated membrane recruitment of dynamin-2 mediated by sorting nexin 9. J Biol Chem 279, 42694-42702 (2004).
19. Soulet, F., Yarar, D., Leonard, M. & Schmid, S.L. SNX9 regulates dynamin assembly and is required for efficient clathrin-mediated endocytosis. Mol Biol Cell 16, 2058-2067 (2005).
20. Shin, N. et al. Sorting nexin 9 interacts with dynamin 1 and N-WASP and coordinates synaptic vesicle endocytosis. J Biol Chem 282, 28939-28950 (2007).
21. Badour, K. et al. Interaction of the Wiskott-Aldrich syndrome protein with sorting nexin 9 is required for CD28 endocytosis and cosignaling in T cells. Proc Natl Acad Sci U S A 104, 1593-1598 (2007).
22. MaCaulay, S.L. et al. Insulin stimulates movement of sorting nexin 9 between cellular compartments: a putative role mediating cell surface receptor expression and insulin action. Biochem J 376, 123-134 (2003).
23. Haberg, K., Lundmark, R. & Carlsson, S.R. SNX18 is an SNX9 paralog that acts as a membrane tubulator in AP-1-positive endosomal trafficking. Journal of Cell Science 121, 1495-1505 (2008).
24. Wang, J.T. et al. The SNX-PX-BAR family in macropinocytosis: the regulation of macropinosome formation by SNX-PX-BAR proteins. PLoS One 5, e13763.
25. Goto, H. & Lindoso, J.A. Immunity and immunosuppression in experimental visceral leishmaniasis. Braz J Med Biol Res 37, 615-623 (2004).
26. Goldberger, O. et al. Exuberated numbers of tumor-specific T cells result in tumor escape. Cancer Res 68, 3450-3457 (2008).
27. Liu, X.P. et al. Anti-tumor activity of N-trimethyl chitosan-encapsulated camptothecin in a mouse melanoma model. J Exp Clin Cancer Res 29, 76.
28. Popivanova, B.K. et al. Blocking TNF-alpha in mice reduces colorectal carcinogenesis associated with chronic colitis. J Clin Invest 118, 560-570 (2008).
29. Nedvetzki, S. et al. CD44 involvement in experimental collagen-induced arthritis (CIA). J Autoimmun 13, 39-47 (1999).
30. Vincent, J. et al. 5-Fluorouracil selectively kills tumor-associated myeloid-derived suppressor cells resulting in enhanced T cell-dependent antitumor immunity. Cancer Res 70, 3052-3061.

## Claims

1. A diagnostic and prognostic method for detecting and monitoring chronic inflammation and associated immune-suppression in a mammalian subject, which method comprises the step of determining the level of expression of SNX9 (sorting nexin 9 protein) in a biological sample of said subject to obtain an expression value, wherein a lower expression value as compared to a predetermined standard expression value or to an expression value of SNX9 in a control sample, is indicative of a chronic inflammation and associated immune-suppression in said subject.

2. The diagnostic and prognostic method according to claim 1, wherein determining the level of expression of SNX9 in a biological sample of said subject is performed by the steps of:
(a) contacting detecting molecules specific for SNX9 (sorting nexin 9 protein) with a biological sample of said subject, or with any protein or nucleic acid product obtained therefrom, and optionally, with a control sample or with any protein or nucleic acid product obtained therefrom;
(b) contacting detecting molecules specific for at least one reference control, with a biological sample of said subject or with any protein or nucleic acid product obtained therefrom, and optionally, with a control sample or with any protein or nucleic acid product obtained therefrom;
(c) determining the expression value of SNX9 in said biological sample according to step (a), and optionally, in said suitable control sample;
(d) determining the expression value of said at least one reference control in said biological sample according to step (b), and optionally, in said suitable control sample;
(e) comparing the normalized SNX9 expression value in said biological sample obtained in step (c), with a predetermined standard expression value or with a expression value of SNX9 in said control sample optionally obtained in step (c);
wherein a lower expression value as compared to a predetermined standard expression value or to the expression value of SNX9 in a control sample, is indicative of a chronic inflammation and associated immune-suppression in said subject.

3. The method according to claim 1, wherein said method further comprises the step of at least one of:
(a) determining the level of expression of SNX18 (sorting nexin 18 protein) in a biological sample of said subject to obtain an expression value;
(b) determining the level of expression of T cell antigen receptor (TCR) ζ chain in a biological sample of said subject, to obtain an expression value; and
(c) determining myeloid-derived suppressor cells (MDSCs) population in a biological sample of said subject;
wherein a lower expression value of SNX9 and of at least one of SNX18 and TCR ζ chain as compared to a predetermined standard expression values or to the expression values of said SNX9 and of at least one of SNX18 and TCR ζ chain in a control sample, and optionally, an enlarged population of MDSCs, indicate a chronic inflammation and associated immune-suppression in said subject.

4. The method according to claim 2, wherein said detecting molecules are selected
from isolated detecting amino acid molecules and isolated detecting nucleic acid
molecules, said detecting amino acid molecule is an isolated antibody that specifically recognizes and binds SNX9.

5. The method according to claim 2, wherein said reference control is at least one of CD3ε, CD3δ, CD3γ, TCRα, TCRβ, CD19 and SNX27.

6. The method according to claim 1, wherein said subject is suffering from a chronic inflammatory condition, said chronic inflammatory condition is any one of an autoimmune disease, a proliferative disorder and an infectious disease.

7. The method according to claim 1, wherein said biological sample is a blood sample, cells from lymph nodes and spleen and tissue biopsies.

8. The method according to claim 1, for the diagnosis, prognosis, evaluating the immune status, and monitoring the effect of therapy in subjects suffering of a chronic inflammatory condition.

9. The diagnostic method for evaluating the efficacy of a treatment with a therapeutic agent on a subject suffering from a chronic-inflammatory condition, which method comprises the steps of:
(a) determining the level of expression of SNX9 in a biological sample of said subject, to obtain SNX9 expression value in said sample, wherein said sample is obtained prior to initiation of said treatment;
(b) determining the level of expression of SNX9 in at least one other biological sample of said subject, to obtain SNX9 expression value in said sample, wherein said at least one other sample is obtained after initiation of said treatment;
(c) comparing SNX9 expression value in said biological sample obtained in step (a), with at least one SNX9 expression value obtained in step (b);
wherein a higher SNX9 expression value in a sample obtained after initiation of said treatment according to step (b) as compared to the SNX9 expression value in a sample obtained prior to initiation of said treatment according to step (a), is indicative of successful therapy.

10. The diagnostic method according to claim 2, for monitoring and assessing responsiveness of a subject suffering from a chronic-inflammatory condition to a treatment with a therapeutic agent, said method further comprising the steps of:
(f) repeating steps (a) to (e) to obtain expression values of said SNX9, for at least one more temporally-separated test sample, wherein a first sample is obtained prior to initiation of said treatment, and at least one more temporally-separated test sample is obtained after the initiation of said treatment; and
(g) calculating the rate of change of said expression values of said SNX9 between said temporally-separated test samples;
wherein a positive rate of change of said expression values in a sample obtained after initiation of said treatment as compared to the SNX9 expression value in a sample obtained prior to initiation of said treatment, is indicative of the responsiveness of said subject to said anti-inflammatory treatment.

11. A diagnostic kit for detecting and monitoring chronic inflammation and associated immune-suppression in a mammalian subject, comprising:
(a) detecting molecules specific for determining the level of expression of SNX9 in a biological sample;
(b) detecting molecules specific for determining the level of expression of at least one control reference in a biological sample,
further comprising:
- detecting molecules specific for determining the level of expression of SNX18 in a biological sample;
- detecting molecules specific for determining the level of expression of TCR ζ chain in a biological sample; and
- detecting molecules for determining MDSCs population in a biological sample;
wherein said detecting molecule is at least one of an isolated antibody that specifically recognizes and binds SNX18, an isolated antibody that specifically recognizes and binds TCR ζ chain, an isolated antibody that specifically recognizes and binds CD11b and an isolated antibody that specifically recognizes and binds Gr1.

## Patentansprüche

1. Diagnostisches und prognostisches Verfahren zum Detektieren und Überwachen von chronischer Entzündung und assoziierter Immunsuppression bei einem Säugetiersubjekt, wobei das Verfahren den Schritt umfasst, das Expressionsniveau von SNX9 (Sortierungs-Nexin-9-Protein) in einer biologischen Probe des Subjekts zu bestimmen, um einen Expressionswert zu gewinnen, wobei ein niedrigerer Expressionswert im Vergleich mit einem vorbestimmten Standardexpressionswert oder mit einem Expressionswert von SNX9 in einer Kontrollprobe eine chronische Entzündung und assoziierte Immunsuppression bei dem Subjekt anzeigt.

2. Diagnostisches und prognostisches Verfahren gemäß Anspruch 1, wobei das Bestimmen des Expressionsniveaus von SNX9 in einer biologischen Probe des Subjekts durch die Schritte erfolgt:
(a) Kontaktieren von Detektionsmolekülen, die für SNX9 (Sortierungs-Nexin-9-Protein) spezifisch sind, mit einer biologischen Probe des Subjekts oder mit jedem daraus gewonnenen Protein oder Nukleinsäureprodukt, und optional mit einer Kontrollprobe oder mit jedem daraus gewonnenen Protein oder Nukleinsäureprodukt;
(b) Kontaktieren von Detektionsmolekülen, die für mindestens eine Referenzkontrolle spezifisch sind, mit einer biologischen Probe des Subjekts oder mit jedem daraus gewonnenen Protein oder Nukleinsäureprodukt, und optional mit einer Kontrollprobe oder mit jedem daraus gewonnenen Protein oder Nukleinsäureprodukt;
(c) Bestimmen des Expressionswerts von SNX9 in der biologischen Probe gemäß Schritt (a) und optional in der geeigneten Kontrollprobe;
(d) Bestimmen des Expressionswerts der mindestens einen Referenzkontrolle in der biologischen Probe gemäß Schritt (b) und optional in der geeigneten Kontrollprobe;
(e) Vergleichen des normalisierten SNX9-Expressionswerts in der in Schritt (c) gewonnenen biologischen Probe mit einem vorbestimmten Standardexpressionswert oder mit einem Expressionswert von SNX9 in der optional in Schritt (c) gewonnenen Kontrollprobe;
wobei ein niedrigerer Expressionswert im Vergleich mit einem vorbestimmten Standardexpressionswert oder mit dem Expressionswert von SNX9 in einer Kontrollprobe eine chronische Entzündung und assoziierte Immunsuppression bei dem Subjekt anzeigt.

3. Verfahren gemäß Anspruch 1, wobei das Verfahren weiter mindestens einen der Schritte umfasst:
(a) Bestimmen des Expressionsniveaus von SNX18 (Sortierungs-Nexin-18-Protein) in einer biologischen Probe des Subjekts, um einen Expressionswert zu gewinnen;
(b) Bestimmen des Expressionsniveaus von T-Zell-Antigen-Rezeptor- (TCR-) ζ-Kette in einer biologischen Probe des Subjekts, um einen Expressionswert zu gewinnen; und
(c) Bestimmen einer Population knochenmarksabgeleiteter Suppressorzellen (MDSCs) in einer biologischen Probe des Subjekts;
wobei ein niedrigerer Expressionswert von SNX9 und von mindestens entweder SNX18 oder TCR-ζ-Kette im Vergleich mit vorbestimmten Standardexpressionswerten oder mit den Expressionswerten des SNX9 und von mindestens entweder SNX18 oder TCR-ζ-Kette in einer Kontrollprobe und optional eine vergrößerte Population von MDSCs eine chronische Entzündung und assoziierte Immunsuppression bei dem Subjekt anzeigen.

4. Verfahren gemäß Anspruch 2, wobei die Detektionsmoleküle aus isolierten Detektions-Aminosäuremolekülen und isolierten Detektions-Nukleinsäuremolekülen ausgewählt sind, wobei das Detektions-Aminosäuremolekül ein isolierter Antikörper ist, der spezifisch SNX9 erkennt und bindet.

5. Verfahren gemäß Anspruch 2, wobei die Referenzkontrolle mindestens entweder CD3ε, CD3δ, CD3γ, TCRα, TCRβ, CD19 oder SNX27 ist.

6. Verfahren gemäß Anspruch 1, wobei das Subjekt an einer chronischen entzündlichen Störung leidet, wobei die chronische entzündliche Störung eines von einer Autoimmunerkrankung, einer proliferativen Störung und einer infektiösen Erkrankung ist.

7. Verfahren gemäß Anspruch 1, wobei die biologische Probe eine Blutprobe, Zellen aus Lymphknoten und Milz und Gewebebiopsien sind.

8. Verfahren gemäß Anspruch 1 zur Diagnose, Prognose, Bewertung des Immunstatus und Überwachung der Wirkung einer Therapie bei Subjekten, die an einer chronischen entzündlichen Störung leiden.

9. Diagnostisches Verfahren zur Bewertung der Wirksamkeit einer Behandlung mit einem therapeutischen Mittel an einem Subjekt, das an einer chronisch-entzündlichen Störung leidet, wobei das Verfahren die Schritte umfasst:
(a) Bestimmen des Expressionsniveaus von SNX9 in einer biologischen Probe des Subjekts, um einen SNX9-Expressionswert in der Probe zu gewinnen, wobei die Probe vor Beginn der Behandlung gewonnen ist;
(b) Bestimmen des Expressionsniveaus von SNX9 in mindestens einer weiteren biologischen Probe des Subjekts, um einen SNX9-Expressionswert in der Probe zu gewinnen, wobei die mindestens eine weitere Probe nach Beginn der Behandlung gewonnen ist;
(c) Vergleichen des SNX9-Expressionswerts in der in Schritt (a) gewonnenen biologischen Probe mit mindestens einem in Schritt (b) gewonnenen SNX9-Expressionswert;
wobei ein höherer SNX9-Expressionswert in einer nach Beginn der Behandlung gewonnen Probe gemäß Schritt (b) im Vergleich mit dem SNX9-Expressionswert in einer vor Beginn der Behandlung gewonnenen Probe gemäß Schritt (a) eine erfolgreiche Therapie anzeigt.

10. Diagnostisches Verfahren gemäß Anspruch 2 zum Überwachen und Beurteilen des Ansprechens eines Subjekts, das an einer chronisch-entzündlichen Störung leidet, auf eine Behandlung mit einem therapeutischen Mittel, wobei das Verfahren weiter die Schritte umfasst:
(f) Wiederholen der Schritte (a) bis (e), um Expressionswerte des SNX9 zu gewinnen, für mindestens eine weitere, zeitlich getrennte Testprobe, wobei eine erste Probe vor Beginn der Behandlung gewonnen ist und mindestens eine weitere, zeitlich getrennte Testprobe nach Beginn der Behandlung gewonnen ist; und
(g) Berechnen der Veränderungsrate der Expressionswerte des SNX9 zwischen den zeitlich getrennten Testproben;
wobei eine positive Veränderungsrate der Expressionswerte in einer Probe, die nach Beginn der Behandlung gewonnen ist, im Vergleich mit dem SNX9-Expressionswert in einer Probe, die vor Beginn der Behandlung gewonnen ist, das Ansprechen des Subjekts auf die entzündungshemmende Behandlung anzeigt.

11. Diagnose-Kit zum Detektieren und Überwachen von chronischer Entzündung und assoziierter Immunsuppression bei einem Säugetiersubjekt, umfassend:
(a) Detektionsmoleküle, die zur Bestimmung des Expressionsniveaus von SNX9 in einer biologischen Probe spezifisch sind;
(b) Detektionsmoleküle, die zur Bestimmung des Expressionsniveaus mindestens einer Kontrollreferenz in einer biologischen Probe spezifisch sind,
weiter umfassend:
- Detektionsmoleküle, die zur Bestimmung des Expressionsniveaus von SNX18 in einer biologischen Probe spezifisch sind;
- Detektionsmoleküle, die zur Bestimmung des Expressionsniveaus von TCR-ζ-Kette in einer biologischen Probe spezifisch sind; und
- Detektionsmoleküle zur Bestimmung einer Population von MDSCs in einer biologischen Probe;
wobei das Detektionsmolekül mindestens entweder ein isolierter Antikörper, der spezifisch SNX18 erkennt und bindet, ein isolierter Antikörper, der spezifisch TCR-ζ-Kette erkennt und bindet, ein isolierter Antikörper, der spezifisch CD11b erkennt und bindet, oder ein isolierter Antikörper, der spezifisch Gr1 erkennt und bindet, ist.

## Revendications

1. Méthode de diagnostic et de prognostique en vue de la détection et de la surveillance d'une inflammation chronique et de la suppression immunitaire associée chez un sujet mammifère, ladite méthode comprenant l'étape de détermination du niveau d'expression de SNX9 (protéine de nexine 9 de classification) dans un échantillon biologique dudit sujet afin d'obtenir une valeur d'expression, dans laquelle une valeur d'expression plus basse que comparée à une valeur d'expression standard prédéterminée ou à une valeur d'expression de SNX9 dans un échantillon de contrôle, est indicatrice d'une inflammation chronique et de la suppression immunitaire associée chez ledit sujet.

2. Méthode de diagnostic et de pronostique selon la revendication 1, dans laquelle la détermination du niveau d'expression de SNX9 dans un échantillon biologique dudit sujet est réalisée par les étapes de :
(a) mise en contact en détectant les molécules spécifiques de SNX9 (protéine de nexine 9 de classification) avec un échantillon biologique dudit sujet ou avec une quelconque protéine ou un produit d'acide nucléique obtenu à partir de celui-ci, et en option, avec un échantillon de contrôle ou avec une quelconque protéine ou un produit d'acide nucléique obtenu à partir de celui-ci ;
(b) mise en contact en détectant les molécules spécifiques pour au moins un contrôle de référence avec un échantillon biologique dudit sujet ou avec une quelconque protéine ou produit d'acide nucléique obtenu à partir de celui-ci, et en option, avec un échantillon de contrôle ou avec une quelconque protéine ou un produit d'acide nucléique obtenu à partir de celui-ci ;
(c) détermination de la valeur d'expression de SNX9 dans ledit échantillon biologique selon l'étape (a), et en option, dans ledit échantillon de contrôle ;
(d) détermination de la valeur d'expression dudit au moins un contrôle de référence dans ledit échantillon biologique selon l'étape (b), et en option, dans ledit échantillon de contrôle ;
(e) comparaison de la valeur d'expression de SNX9 dans ledit échantillon biologique obtenu à l'étape (c), avec une valeur d'expression standard prédéterminée ou avec une valeur d'expression de SNX9 dans ledit échantillon de contrôle obtenu en option à l'étape (c) ;
dans laquelle une valeur d'expression plus basse que comparée à une valeur d'expression standard prédéterminée ou à la valeur d'expression de SNX9 dans un échantillon de contrôle est indicatrice d'une inflammation chronique et de la suppression immunitaire associée chez ledit sujet.

3. Méthode selon la revendication 2, dans laquelle ladite méthode comprend au moins l'étape de:
(a) détermination du niveau d'expression de SNX18 (protéine de nexine 18 de classification) dans un échantillon biologique dudit sujet afin d'obtenir une valeur d'expression ;
(b) détermination du niveau d'expression d'une chaîne (TCR) ζ de récepteur antigène de cellule T dans un échantillon biologique dudit sujet afin d'obtenir une valeur d'expression ; et
(c) détermination de la population de cellules suppressives dérivées de myéloïde (MDSC) dans un échantillon biologique dudit sujet ;
dans laquelle une valeur d'expression plus basse de SNX9 et d'au moins l'une de la SNX18 et de la chaîne TCR ζ telle que comparée à une valeur d'expression standard prédéterminée ou aux valeurs d'expression de ladite SNX9 et d'au moins l'une de la SNX18 et de la chaîne TCR ζ dans un échantillon de contrôle, et en option, une population élargie de MDSC, indiquent une inflammation chronique et la suppression immunitaire associée chez ledit sujet.

4. Méthode selon la revendication 2, dans laquelle lesdites molécules de détection sont sélectionnées à partir de : molécules de détection isolées d'acide aminé et de molécules de détection isolées d'acide nucléique, ladite molécule de détection isolée d'acide aminé est un anticorps isolé qui reconnaît spécifiquement et lie le SNX9.

5. Méthode selon la revendication 2, dans laquelle ledit contrôle de référence est au moins un de CD3ε, CD3δ, CD3γ, TCRα, TCRβ, CD19 et SNX27.

6. Méthode selon la revendication 1, dans laquelle ledit sujet souffre d'un état inflammatoire chronique, ledit état inflammatoire chronique est un quelconque d'une maladie auto-immune, d'un trouble prolifératif et d'une maladie infectieuse.

7. Méthode selon la revendication 1, dans laquelle ledit échantillon biologique est un échantillon sanguin, des cellules des ganglions lymphatiques, de la rate et des biopsies des tissus.

8. Méthode selon la revendication 1, en vue du diagnostic, du pronostique en évaluant le statut immunitaire et en surveillant l'effet de la thérapie chez les sujets souffrant d'un état inflammatoire chronique.

9. Méthode de diagnostic en vue de l'évaluation de l'efficacité d'un traitement avec un agent thérapeutique sur un sujet souffrant d'un état inflammatoire chronique, ladite méthode comprenant les étapes de :
(a) détermination du niveau d'expression de SNX9 dans un échantillon biologique dudit sujet, afin d'obtenir une valeur d'expression de SNX9 dans ledit échantillon, dans laquelle ledit échantillon est obtenu avant le lancement dudit traitement ;
(b) détermination du niveau d'expression de SNX9 dans au moins un autre échantillon biologique dudit sujet, afin d'obtenir une valeur d'expression de SNX9 dans ledit échantillon, dans laquelle au moins un autre échantillon est obtenu après le lancement dudit traitement ;
(c) comparaison de la valeur d'expression de SNX9 dans ledit échantillon biologique obtenu à l'étape (a), avec au moins une valeur d'expression obtenue à l'étape (b) ;
dans laquelle une plus haute valeur d'expression de SNX9 dans un échantillon obtenu après le lancement dudit traitement selon l'étape (b) telle que comparée à la valeur d'expression de SNX9 dans un échantillon obtenu avant le lancement dudit traitement selon l'étape (a), est indicatrice d'une thérapie réussie.

10. Méthode de diagnostic selon la revendication 2, en vue de la surveillance et de l'évaluation de la réactivité d'un sujet souffrant d'un état inflammatoire chronique suite à un traitement avec un agent thérapeutique, ladite méthode comprenant par ailleurs les étapes de :
(f) répétition des étapes (a) à (e) afin d'obtenir des valeurs d'expression de ladite SNX9, pour au moins un échantillon de test séparé temporellement supplémentaire, dans laquelle un premier échantillon est obtenu avant le lancement dudit traitement, et au moins un échantillon de test séparé temporellement supplémentaire est obtenu après le lancement dudit traitement ; et
(g) calcul du taux de variation desdites valeurs d'expression de ladite SNX9 entre lesdits échantillons de test séparés temporellement ;
dans lequel un taux de variation positif desdites valeurs d'expression dans un échantillon obtenu après le lancement dudit traitement tel que comparé à la valeur d'expression de SNX9 dans un échantillon obtenu avant le lancement dudit traitement, est indicateur de la réactivité dudit sujet audit traitement inflammatoire.

11. Kit diagnostic en vue de la détection et de la surveillance d'une inflammation chronique et de la suppression immunitaire associée chez un sujet mammifère, comprenant :
(a) la détection de molécules spécifiques en vue de la détermination du niveau d'expression de SNX9 dans un échantillon biologique;
(b) la détection de molécules spécifiques en vue de la détermination du niveau d'expression d'au moins une référence de contrôle dans un échantillon biologique,
comprenant par ailleurs :
- la détection de molécules spécifiques en vue de la détermination du niveau d'expression de SNX18 dans un échantillon biologique ;
- la détection de molécules spécifiques en vue de la détermination du niveau d'expression de la chaîne TCR ζ dans un échantillon biologique ; et
- la détection de molécules en vue de la détermination d'une population de MDSC dans un échantillon biologique ;
dans lequel ladite molécule de détection est au moins un d'un anticorps isolé qui qui reconnaît spécifiquement et lie le SNX18, d'un anticorps isolé qui qui reconnaît spécifiquement et lie la chaîne de TCRζ, d'un anticorps isolé qui qui reconnaît spécifiquement et lie le CD11b et d'un anticorps isolé qui qui reconnaît spécifiquement et lie le Gr1.


### SEQUENCE LISTING

<110> Yissum
<120> SNX9 AS A NOVEL BIOMARKER FOR CHRONIC INFLAMMATION AND ASSOCIATED IMMUNOSUPPRESSION AND A NEW REGULATOR OF T CELL RECEPTOR EXPRESSION AND FUNCTION
<130> 29395-WO-11
<160> 2
<170> PatentIn version 3.5
<210> 1
   <211> 22
   <212> DNA
   <213> Mouse
<400> 1
   aacagtgtga tccgcctcta cc 22
<210> 2
   <211> 23
   <212> DNA
   <213> Mouse
<400> 2
   tgaagcactt cggagttctc ttc 23